# EUROPÄISCHE PATENTSCHRIFT

(11) **EP 0 781 343 B1**
(45) Veröffentlichungstag und Bekanntmachung des Hinweises auf die Patenterteilung: **02.07.2003**
(21) Anmeldenummer: 95929100.6
(22) Anmeldetag: 10.08.1995
(51) Int. Cl.: C12N 15/86, C12N 7/01, C12N 5/10, A61K 48/00

(54) **RETROVIRALE VEKTORHYBRIDE UND DEREN VERWENDUNG ZUM GENTRANSFER**
RETROVIRAL VECTOR HYBRIDS AND THE USE THEREOF FOR GENE TRANSFER
HYBRIDES VECTORIELS RETROVIRAUX ET LEUR UTILISATION POUR LE TRANSFERT DE GENES

(30) Priorität: 08.09.1994 DE 4431973; 07.02.1995 DE 19503952
(43) Veröffentlichungstag der Anmeldung: 02.07.1997
(73) Patentinhaber: Vision 7 GmbH, 20149 Hamburg (DE)
(72) Erfinder: BAUM, Christopher, D-22589 Hamburg (DE); STOCKING-HARBERS, Carol, D-20249 Hamburg (DE); OSTERTAG, Wolfram, D-22397 Hamburg (DE)
(74) Vertreter: UEXKÜLL & STOLBERG
(86) Internationale Anmeldenummer: EP9503175
(87) Internationale Veröffentlichungsnummer: WO96007747

(56) Entgegenhaltungen:
- WO-A-94/09120
- WO-A-94/13824
- PROC. NATL.ACAD SCI., Bd. 80, Juli 1983 NATL. ACAD SCI.,WASHINGTON,DC,US;, Seiten 4408-4411, P.A. CHATIS ET AL. 'Role for the 3' end of the genome in the determining disease specificty of Friend and Moloney murine leukemia virus'
- PROC. NATL.ACAD SCI., Bd. 87, Dezember 1990 NATL. ACAD SCI.,WASHINGTON,DC,US;, Seiten 9202-9206, M. GREZ ET AL. 'Embryonic stem cell virus, a recombinant murine retrovirus with expression in embyonic stem cells' in der Anmeldung erwähnt
- J. VIROLOGY, Bd. 61, Nr. 3, März 1987 AM.SOC.MICROBIOL.,WASHINGTON,US, Seiten 693-700, Y. LI ET AL. 'Disease specificity of non defective Friend and Moleney murine leukemia viruses is controlled by a small number of nucleotides'
- COHEN-HAGUENAUER, O. AND M. BOIRON (ED.). COLLOQUE INSERM (INSTITUT NATIONAL DE LA SANTE ET DE LA RECHERCHE MEDICALE), VOL. 219. TRANSFERT DE GENES CHEZ L'HOMME;(INSERM (NATIONAL INSTITUTE OF HEALTH AND MEDICAL RESEARCH) COLLOQUIUM), VOL. 219. HUMAN , 1991 Seiten 273-274, VELU T J ET AL 'HIGHLY EFFICIENT RETROVIRAL VECTORS DERIVED FROM HARVEY AND FRIEND MURINE VIRUSES.'
- VIRUS STRATEGIES, MOLECULAR BIOLOGY AND PATHOGENESIS, ED., W. DOERFLER AND P. BÖHM, 1993 VCH, WEINHEIM, BRD, Seiten 433-455, C.STOCKING ET AL. 'Regulation of retrovirus infection and expression in embryonic and hematopoietic stem cells' in der Anmeldung erwähnt
- MOL. CELL. BIOL., Bd. 10, Nr. 8, August 1990 ASM WASHINGTON, DC,US, Seiten 4045-4057, T.P. LOH ET AL. 'Evidence for a stem cell-specific repressor of Moloney murine leukemia virus expression in embryonal carcinoma cells'
- GENE, Bd. 84, 1989 ELSEVIER SCIENCE PUBLISHERS,B.V.,AMSTERDAM,NL;, Seiten 419-427, D. VALERIO ET AL. 'Retrovirus-mediated gene transfer into embryonal carcinoma and hemopoietic stem cells: expression from a hybrid long terminal repeat'
- J. VIROLOGY, Bd. 65, Nr. 1, Januar 1991 AM.SOC.MICROBIOL.,WASHINGTON,US, Seiten 382-388, E. AKGÜN ET AL. 'Determinants of retrovirus gene expression in ambryonal carcinoma cells'
- HUMAN GENE THERAPY, Bd. 5, Nr. 6, Juni 1994 MARY ANN LIEBERT, INC. PUBLISHERS, NEW YORK, US, Seiten 667-677, L.A. COUTURE ET AL. 'Retroviral vectors containing chimeric promoter/enhancer elements exhibit cell-type-specific gene expression'
- HUMAN GENE THERAPY, Bd. 2, Nr. 1, 1991 MARY ANN LIEBERT, INC. PUBLISHERS, NEW YORK, US, Seiten 61-70, G. BECK-ENGESER ET AL. 'Retroviral vectors related to the Myeloproliferative sarcoma virus allow efficient expression in hematopoietic stem and precursor cell lines, but retroviral infection is reduced in more primitive cells'
- J. VIROLOGY, Bd. 51, Nr. 2, August 1984 AM.SOC.MICROBIOL.,WASHINGTON,US, Seiten 306-314, M.A. GONDA ET AL. 'Heteroduplex analysis of molecular clones of the pathogenic Friend virus complex: Friend murine leukemia virus, friend mink cell focus-forming virus, and the polycythemia- and anemia-inducing strains of Friend spleen focus-forming virus'
- J. VIROLOGY, Bd. 68, Nr. 11, November 1994 AM.SOC.MICROBIOL.,WASHINGTON,US, Seiten 7235-7243, N. AHLERS ET AL. 'Selectable retrovirus vector encoding Friend virus gp55 or erythropoietin induce polycythemia with different phenotypic expression and disease progression'
- 24TH ANNUAL MEETING OF THE INTERNATIONAL SOCIETY FOR EXPERIMENTAL HEMATOLOGY, DUESSELDORF, GERMANY, AUGUST 27-31, 1995. EXPERIMENTAL HEMATOLOGY (CHARLOTTESVILLE) 23 (8). 1995. 841. ISSN: 0301-472X, 30.August 1995 OSTERTAG W ET AL 'Novel and efficient retroviral vectors for somatic gene therapy and for stem cell protection.'

### SEQUENZPROTOKOLL

(1) ALLGEMEINE ANGABEN:
   (i) ANMELDER:
      (A) NAME: BOEHRINGER MANNHEIM GMBH
      (B) STRASSE: Sandhofer Str. 116
      (C) ORT: Mannheim
      (E) LAND: Deutschland
      (F) POSTLEITZAHL: D-69305
      (G) TELEFON: 08856/60-3446
      (H) TELEFAX: 08856/60-3451
   (ii) BEZEICHNUNG DER ERFINDUNG: Retrovirale Vektorhybride und deren Verwendung zum Gentransfer
   (iii) ANZAHL DER SEQUENZEN: 6
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30B (EPA)
   (vi) DATEN DER URANMELDUNG:
      (A) ANMELDENUMMER: DE P 44 31 973.8
      (B) ANMELDETAG: 08-SEP-1994
   (vi) DATEN DER URANMELDUNG:
      (A) ANMELDENUMMER: DE 195 03 952.1
      (B) ANMELDETAG: 07-FEB-1995
(2) INFORMATION ZU SEQ ID NO: 1:
   (i) SEQUENZ KENNZEICHEN:
      (A) LÄNGE: 5323 Basenpaare
      (B) ART: Nucleotid
      (C) STRANGFORM: Doppelstrang
      (D) TOPOLOGIE: ringförmig
   (ii) ART DES MOLEKÜLS: Genom-DNA
   (xi) SEQUENZBESCHREIBUNG: SEQ ID NO: 1:
   CGATTAGTCC AATTTGTTAA AGACAGGATA TCAGGTGGTC CAGGCTCTAG TTTTGACTCA 60
   ACAATATCAC CAGCTGAAGC CTATAGAGTA CGAGCCATAG ATAGAATAAA AGATTTTATT 120
   TAGTCTCCAG AAAAAGGGGG GAATGAAAGA CCCCACCTGT AGGTTTGGCA AGCTAGCTTA 180
   AGTAACGCCA TTTTGCAAGG CATGGAAAAT ACATAACTGA GAATAGAGAA GTTCAGATCA 240
   AGGTTAGGAA CAGAGAGACA GCAGAATATG GGCCAAACAG GATATCTGTG GTAAGCAGTT 300
   CCTGCCCCGC TCAGGGCCAA GAACAGATGG TCCCCAGATG CGGTCCCGCC CTCAGCAGTT 360
   TCTAGAGAAC CATCAGATGT TTCCAGGGTG CCCCAAGGAC CTGAAAATGA CCCTGTGCCT 420
   TATTTGAACT AACCAATCAG TTCGCTTCTC GCTTCTGTTC GCGCGCTTCT GCTCCCCGAG 480
   CTCAATAAAA GAGCCCACAA CCCCTCACTC GGCGCGCCAG TCCTCCGATT GACTGCGTCG 540
   CCCGGGTACC CGTATTCCCA ATAAAGCCTC TTGCTGTTTG CATCCGAATC GTGGACTCGC 600
   TGATCCTTGG GAGGGTCTCC TCAGATTGAT TGACTGCCCA CCTCGGGGGT CTTTCATTTG 660
   GAGGTTCCAC CGAGATTTGG AGACCCCAGC CCAGGGACCA CCGACCCCCC CGCCGGGAGG 720
   TAAGCTGGCC AGCGGTCGTT TCGTGTCTGT CTCTGTCTTT GTGCGTGTTT GTGCCGGCAT 780
   CTAATGTTTG CGCCTGCGTC TGTACTAGTT GGCTAACTAG ATCTGTATCT GGCGGTCCCG 840
   CGGAAGAACT GACGAGTTCG TATTCCCGGC CGCAGCCCCT AGGAGACGTC CCAGCGGCCT 900
   CGGGGGCCCG TTTTGTGGCC CGTTCTGTGT CGTTAACCAC CCGAGTCGGA CTTTTTGGAG 960
   CTCCGCCACT GTCCGAGGGG TACGTGGCTT TGTTGGGGGA CGAGAGACAG AGACACTTCC 1020
   CGCCCCCGTC TGAATTTTTG CTTTCGGTTT TACGCCGAAA CCGCGCCGCG CGTCTTGTCT 1080
   GCTGCAGCAT CGTTCTGTGT TGTCTCTGTC TGACTGTGTT TCTGTATTTG TCTGAAAATT 1140
   AGGGCCAGAC TGTTACCACT CCCTTAAGTT TGACCTTAGA TCACTGGAAA GATGTCGAGC 1200
   GGATCGCTCA CAACCAGTCG GTAGATGTCA AGAAGAGACG ATGGGTTACC TTCTGCTCTG 1260
   CAGAATGGCC AACCTTTAAC GTCGGATGGC CGCGAGACGG CACCTTTAAC CGAGACCTCA 1320
   TCACCCAGGT TAAGATCAAG GTCTTTTCAC CTGGCCCGCA TGGACACCCA GACCAGGTCC 1380
   CCTACATCGT GACCTGGGAA GCCTTGGCTT TTGACCCCCC TCCCTGGGTC AAGCCCTTTG 1440
   TACACCCTAA GCCTCCGCCT CCTCTTCCTC CATCCGCCCC GTCTCTCCCC CTTGAACCTC 1500
   CTCTTTCGAC CCCGCCTCGA TCCTCCCTTT ATCCAGCCCT CACTCCTTCT CTAGGCGGCT 1560
   CCACCGCGGT GGCGGCCGCT CTAGAACTAG TGGATCCAAG CTTATCGATA GGCCTAGGCC 1620
   TATCGATAGG CCTAGGCCTA TCGATAGGCC TAACACGAGC CATAGATAGA ATAAAAGATT 1680
   TTATTTAGTC TCCAGAAAAA GGGGGGAATG AAAGACCCCA CCTGTAGGTT TGGCAAGCTA 1740
   GAGTCGCTTA GCCTGATAGC CGCAGTAACG CCATTTTGCA AGGCATGGAA AAATACCAAA 1800
   CCAAGAATAG GGAAGTTCAG ATCAAGGGCG GGTACATGAA AATAGCTAAC GTTGGGCCAA 1860
   ACAGGATATC TGCGGTGAGC AGTTTCGGCC CCGGCCCGGG GCAAGAACAG ATGGTCACCG 1920
   CAGTTTCGGC CCCGGCCCGA GGCCAAGAAC AGATGGTCCC CAGATATGGC CCAACCCTCA 1980
   GCAGTTTCTT AAGACCCATC AGATGTTTCC AGGCTCCCCC AAGGACCTGA AATGACCCTG 2040
   CGCCTTATTT GAATTAACCA ATCAGCCTGC TTCTCGCTTC TGTTCGCGCG CTTCTGCTTC 2100
   CCGAGCTCTA TAAAAGAGCT CACAACCCCT CACTCGGCGC GCCAGTCCTC CGATTGACTG 2160
   AGTCGCCCGG GTACCCGTGT TCTCAATAAA CCCTCTTGCA GTTGCATCCG ACTCGTGGTC 2220
   TCGCTGTTCC TTGGGAGGGT CTCCTCTGAG TGATTGACTA CCCGTCAGCG GGGGTCTTTC 2280
   AGTTTCTCCC ACCTACACAG GTCTCACTAA CATTCCTGAT GTGCCGCAGG GACTCCGTCA 2340
   GCCCGGTTTG TGTTTATAAT AAAATGCAAG AACAGTGTTC CCTTCAAGCC AGACTACATC 2400
   CTGACTCTCG GCTTTATAAA AGAATGTTGA AGGGCTCTGT GGACTATCTG CCACACGACT 2460
   TTTAAGATTT TTATGCCTCC TGGATGAGGG ATTTAGTCAA TCTATCCTCG TCTATTTTGC 2520
   TGGCTTCTCC GTATTTTAAA TTTCTAGTTT GCACTCCCTT CCTGAGAGCA CGGCGATTGC 2580
   AGAGTAGTTA ATACTCTGAG GGCAGGCTTC TGTGAAAAGG TTGCCTGGGC TCAGTGTGAG 2640
   ATTTTGCCAT AAAAAGGGGT CCTGCCCCTG TGTACAGACA GATCGGAATC TAGAGTGCAT 2700
   ACTCAGAGTC CCCGCGGTTC CGGGGCTCTG ATCTCAGGGC ATCTTTGCCT AGAGATCCTC 2760
   TACGCCGGAC GCATCGTGGC CGGCATCACC GGCGCCACAG GTGCGGTTGC TGGCGCCTAT 2820
   ATCGCCGACA TCACCGATGG GGAAGATCGG GCTCGCCACT TCGGGCTCAT GAGCGCTTGT 2880
   TTCGGCGTGG GTATGGTGGC AGGCCCCGTG GCCGGGGGAC TGTTGGGCGC CATCTCCTTG 2940
   CATGCACCAT TCCTTGCGGC GGCGGTGCTC AACGGCCTCA ACCTACTACT GGGCTGCTTC 3000
   CTAATGCAGG AGTCGCATAA GGGAGAGCGT CCTGCATTAA TGAATCGGCC AACGCGCGGG 3060
   GAGAGGCGGT TTGCGTATTG GGCGCTCTTC CGCTTCCTCG CTCACTGACT CGCTGCGCTC 3120
   GGTCGTTCGG CTGCGGCGAG CGGTATCAGC TCACTCAAAG GCGGTAATAC GGTTATCCAC 3180
   AGAATCAGGG GATAACGCAG GAAAGAACAT GTGAGCAAAA GGCCAGCAAA AGGCCAGGAA 3240
   CCGTAAAAAG GCCGCGTTGC TGGCGTTTTT CCATAGGCTC CGCCCCCCTG ACGAGCATCA 3300
   CAAAAATCGA CGCTCAAGTC AGAGGTGGCG AAACCCGACA GGACTATAAA GATACCAGGC 3360
   GTTTCCCCCT GGAAGCTCCC TCGTGCGCTC TCCTGTTCCG ACCCTGCCGC TTACCGGATA 3420
   CCTGTCCGCC TTTCTCCCTT CGGGAAGCGT GGCGCTTTCT CAATGCTCAC GCTGTAGGTA 3480
   TCTCAGTTCG GTGTAGGTCG TTCGCTCCAA GCTGGGCTGT GTGCACGAAC CCCCCGTTCA 3540
   GCCCGACCGC TGCGCCTTAT CCGGTAACTA TCGTCTTGAG TCCAACCCGG TAAGACACGA 3600
   CTTATCGCCA CTGGCAGCAG CCACTGGTAA CAGGATTAGC AGAGCGAGGT ATGTAGGCGG 3660
   TGCTACAGAG TTCTTGAAGT GGTGGCCTAA CTACGGCTAC ACTAGAAGGA CAGTATTTGG 3720
   TATCTGCGCT CTGCTGAAGC CAGTTACCTT CGGAAAAAGA GTTGGTAGCT CTTGATCCGG 3780
   CAAACAAACC ACCGCTGGTA GCGGTGGTTT TTTTGTTTGC AAGCAGCAGA TTACGCGCAG 3840
   AAAAAAAGGA TCTCAAGAAG ATCCTTTGAT CTTTTCTACG GGGTCTGACG CTCAGTGGAA 3900
   CGAAAACTCA CGTTAAGGGA TTTTGGTCAT GAGATTATCA AAAAGGATCT TCACCTAGAT 3960
   CCTTTTAAAT TAAAAATGAA GTTTTAAATC AATCTAAAGT ATATATGAGT AAACTTGGTC 4020
   TGACAGTTAC CAATGCTTAA TCAGTGAGGC ACCTATCTCA GCGATCTGTC TATTTCGTTC 4080
   ATCCATAGTT GCCTGACTCC CCGTCGTGTA GATAACTACG ATACGGGAGG GCTTACCATC 4140
   TGGCCCCAGT GCTGCAATGA TACCGCGAGA CCCACGCTCA CCGGCTCCAG ATTTATCAGC 4200
   AATAAACCAG CCAGCCGGAA GGGCCGAGCG CAGAAGTGGT CCTGCAACTT TATCCGCCTC 4260
   CATCCAGTCT ATTAATTGTT GCCGGGAAGC TAGAGTAAGT AGTTCGCCAG TTAATAGTTT 4320
   GCGCAACGTT GTTGCCATTG CTACAGGCAT CGTGGTGTCA CGCTCGTCGT TTGGTATGGC 4380
   TTCATTCAGC TCCGGTTCCC AACGATCAAG GCGAGTTACA TGATCCCCCA TGTTGTGCAA 4440
   AAAAGCGGTT AGCTCCTTCG GTCCTCCGAT CGTTGTCAGA AGTAAGTTGG CCGCAGTGTT 4500
   ATCACTCATG GTTATGGCAG CACTGCATAA TTCTCTTACT GTCATGCCAT CCGTAAGATG 4560
   CTTTTCTGTG ACTGGTGAGT ACTCAACCAA GTCATTCTGA GAATAGTGTA TGCGGCGACC 4620
   GAGTTGCTCT TGCCCGGCGT CAATACGGGA TAATACCGCG CCACATAGCA GAACTTTAAA 4680
   AGTGCTCATC ATTGGAAAAC GTTCTTCGGG GCGAAAACTC TCAAGGATCT TACCGCTGTT 4740
   GAGATCCAGT TCGATGTAAC CCACTCGTGC ACCCAACTGA TCTTCAGCAT CTTTTACTTT 4800
   CACCAGCGTT TCTGGGTGAG CAAAAACAGG AAGGCAAAAT GCCGCAAAAA AGGGAATAAG 4860
   GGCGACACGG AAATGTTGAA TACTCATACT CTTCCTTTTT CAATATTATT GAAGCATTTA 4920
   TCAGGGTTAT TGTCTCATGA GCGGATACAT ATTTGAATGT ATTTAGAAAA ATAAACAAAT 4980
   AGGGGTTCCG CGCACATTTC CCCGAAAAGT GCCACCTGAC GTCTAAGAAA CCATTATTAT 5040
   CATGACATTA ACCTATAAAA ATAGGCGTAT CACGAGGCCC TTTCGTCTCG CGCGTTTCGG 5100
   TGATGACGGT GAAAACCTCT GACACATGCA GCTCCCGGAG ACGGTCACAG CTTGTCTGTA 5160
   AGCGGATGCC GGGAGCAGAC AAGCCCGTCA GGGCGCGTCA GCGGGTGTTG GCGGGTGTCG 5220
   GGGCTGGCTT AACTATGCGG CATCAGAGCA GATTGTACTG AGAGTGCACC ATATGCGGTG 5280
   TGAAATACCG CACAGATGCG TAAGGAGAAA ATACCGCATC AGG 5323
(2) INFORMATION ZU SEQ ID NO: 2:
   (i) SEQUENZKENNZEICHEN:
      (A) LÄNGE: 5294 Basenpaare
      (B) ART: Nucleotid
      (C) STRANGFORM: Doppelstrang
      (D) TOPOLOGIE: ringförmig
   (ii) ART DES MOLEKÜLS: Genom-DNA
   (xi) SEQUENZBESCHREIBUNG: SEQ ID NO: 2:
   CGATTAGTCC AATTTGTTAA AGACAGGATA TCAGGTGGTC CAGGCTCTAG TTTTGACTCA 60
   ACAATATCAC CAGCTGAAGC CTATAGAGTA CGAGCCATAG ATAGAATAAA AGATTTTATT 120
   TAGTCTCCAG AAAAAGGGGG GAATGAAAGA CCCCACCTGT AGGTTTGGCA AGCTAGCTTA 180
   AGTAACGCCA TTTTGCAAGG CATGGAAAAT ACATAACTGA GAATAGAGAA GTTCAGATCA 240
   AGGTTAGGAA CAGAGAGACA GCAGAATATG GGCCAAACAG GATATCTGTG GTAAGCAGTT 300
   CCTGCCCCGC TCAGGGCCAA GAACAGATGG TCCCCAGATG CGGTCCCGCC CTCAGCAGTT 360
   TCTAGAGAAC CATCAGATGT TTCCAGGGTG CCCCAAGGAC CTGAAAATGA CCCTGTGCCT 420
   TATTTGAACT AACCAATCAG TTCGCTTCTC GCTTCTGTTC GCGCGCTTCT GCTCCCCGAG 480
   CTCAATAAAA GAGCCCACAA CCCCTCACTC GGCGCGCCAG TCCTCCGATT GACTGCGTCG 540
   CCCGGGTACC CGTATTCCCA ATAAAGCCTC TTGCTGTTTG CATCCGAATC GTGGACTCGC 600
   TGATCCTTGG GAGGGTCTCC TCAGATTGAT TGACTGCCCA CCTCGGGGGT CTTTCATTTG 660
   GAGGTTCCAC CGAGATTTGG AGACCCCAGC CCAGGGACCA CCGACCCCCC CGCCGGGAGG 720
   TAAGCTGGCC AGCAACTTAT CTGTGTCTGT CCGATTGTCT AGTGTCTATG TTTGATGTTA 780
   TGCGCCTGCG TCTGTACTAG TTAGCTAACT AGCTCTGTAT CTGGCGGACC CGTGGTGGAA 840
   CTGACGAGTT CTGAACACCC GGCCGCAACC CTGGGAGACG TCCCAGGGAC TTTGGGGGCC 900
   GTTTTTGTGG CCCGACCTGA GGAAGGGAGT CGATGTGGAA TCCGACCCCG TCAGGATATG 960
   TGGTTCTGGT AGGAGACGAG AACCTAAAAC AGTTCCCGCC TCCGTCTGAA TTTTTGCTTT 1020
   CGGTTTGGAA CCGAAGCCGC GCGTCTTGTC TGCTGCAGCA TCGTTCTGTG TTGTCTCTGT 1080
   CTGACTGTGT TTCTGTATTT GTCTGAAAAT TAGGGCCAGA CTGTTACCAC TCCCTTAAGT 1140
   TTGACCTTAG ATCACTGGAA AGATGTCGAG CGGATCGCTC ACAACCAGTC GGTAGATGTC 1200
   AAGAAGAGAC GATGGGTTAC CTTCTGCTCT GCAGAATGGC CAACCTTTAA CGTCGGATGG 1260
   CCGCGAGACG GCACCTTTAA CCGAGACCTC ATCACCCAGG TTAAGATCAA GGTCTTTTCA 1320
   CCTGGCCCGC ATGGACACCC AGACCAGGTC CCCTACATCG TGACCTGGGA AGCCTTGGCT 1380
   TTTGACCCCC CTCCCTGGGT CAAGCCCTTT GTACACCCTA AGCCTCCGCC TCCTCTTCCT 1440
   CCATCCGCCC CGTCTCTCCC CCTTGAACCT CCTCTTTCGA CCCCGCCTCG ATCCTCCCTT 1500
   TATCCAGCCC TCACTCCTTC TCTAGGCGGC TCCACCGCGG TGGCGGCCGC TCTAGAACTA 1560
   GTGGATCCAA GCTTATCGAT AGGCCTAGGC CTATCGATAG GCCTAGGCCT ATCGATAGGC 1620
   CTAACACGAG CCATAGATAG AATAAAAGAT TTTATTTAGT CTCCAGAAAA AGGGGGGAAT 1680
   GAAAGACCCC ACCTGTAGGT TTGGCAAGCT AGAGTCGCTT AGCCTGATAG CCGCAGTAAC 1740
   GCCATTTTGC AAGGCATGGA AAAATACCAA ACCAAGAATA GGGAAGTTCA GATCAAGGGC 1800
   GGGTACATGA AAATAGCTAA CGTTGGGCCA AACAGGATAT CTGCGGTGAG CAGTTTCGGC 1860
   CCCGGCCCGG GGCAAGAACA GATGGTCACC GCAGTTTCGG CCCCGGCCCG AGGCCAAGAA 1920
   CAGATGGTCC CCAGATATGG CCCAACCCTC AGCAGTTTCT TAAGACCCAT CAGATGTTTC 1980
   CAGGCTCCCC CAAGGACCTG AAATGACCCT GCGCCTTATT TGAATTAACC AATCAGCCTG 2040
   CTTCTCGCTT CTGTTCGCGC GCTTCTGCTT CCCGAGCTCT ATAAAAGAGC TCACAACCCC 2100
   TCACTCGGCG CGCCAGTCCT CCGATTGACT GAGTCGCCCG GGTACCCGTG TTCTCAATAA 2160
   ACCCTCTTGC AGTTGCATCC GACTCGTGGT CTCGCTGTTC CTTGGGAGGG TCTCCTCTGA 2220
   GTGATTGACT ACCCGTCAGC GGGGGTCTTT CAGTTTCTCC CACCTACACA GGTCTCACTA 2280
   ACATTCCTGA TGTGCCGCAG GGACTCCGTC AGCCCGGTTT GTGTTTATAA TAAAATGCAA 2340
   GAACAGTGTT CCCTTCAAGC CAGACTACAT CCTGACTCTC GGCTTTATAA AAGAATGTTG 2400
   AAGGGCTCTG TGGACTATCT GCCACACGAC TTTTAAGATT TTTATGCCTC CTGGATGAGG 2460
   GATTTAGTCA ATCTATCCTC GTCTATTTTG CTGGCTTCTC CGTATTTTAA ATTTCTAGTT 2520
   TGCACTCCCT TCCTGAGAGC ACGGCGATTG CAGAGTAGTT AATACTCTGA GGGCAGGCTT 2580
   CTGTGAAAAG GTTGCCTGGG CTCAGTGTGA GATTTTGCCA TAAAAAGGGG TCCTGCCCCT 2640
   GTGTACAGAC AGATCGGAAT CTAGAGTGCA TACTCAGAGT CCCCGCGGTT CCGGGGCTCT 2700
   GATCTCAGGG CATCTTTGCC TAGAGATCCT CTACGCCGGA CGCATCGTGG CCGGCATCAC 2760
   CGGCGCCACA GGTGCGGTTG CTGGCGCCTA TATCGCCGAC ATCACCGATG GGGAAGATCG 2820
   GGCTCGCCAC TTCGGGCTCA TGAGCGCTTG TTTCGGCGTG GGTATGGTGG CAGGCCCCGT 2880
   GGCCGGGGGA CTGTTGGGCG CCATCTCCTT GCATGCACCA TTCCTTGCGG CGGCGGTGCT 2940
   CAACGGCCTC AACCTACTAC TGGGCTGCTT CCTAATGCAG GAGTCGCATA AGGGAGAGCG 3000
   TCCTGCATTA ATGAATCGGC CAACGCGCGG GGAGAGGCGG TTTGCGTATT GGGCGCTCTT 3060
   CCGCTTCCTC GCTCACTGAC TCGCTGCGCT CGGTCGTTCG GCTGCGGCGA GCGGTATCAG 3120
   CTCACTCAAA GGCGGTAATA CGGTTATCCA CAGAATCAGG GGATAACGCA GGAAAGAACA 3180
   TGTGAGCAAA AGGCCAGCAA AAGGCCAGGA ACCGTAAAAA GGCCGCGTTG CTGGCGTTTT 3240
   TCCATAGGCT CCGCCCCCCT GACGAGCATC ACAAAAATCG ACGCTCAAGT CAGAGGTGGC 3300
   GAAACCCGAC AGGACTATAA AGATACCAGG CGTTTCCCCC TGGAAGCTCC CTCGTGCGCT 3360
   CTCCTGTTCC GACCCTGCCG CTTACCGGAT ACCTGTCCGC CTTTCTCCCT TCGGGAAGCG 3420
   TGGCGCTTTC TCAATGCTCA CGCTGTAGGT ATCTCAGTTC GGTGTAGGTC GTTCGCTCCA 3480
   AGCTGGGCTG TGTGCACGAA CCCCCCGTTC AGCCCGACCG CTGCGCCTTA TCCGGTAACT 3540
   ATCGTCTTGA GTCCAACCCG GTAAGACACG ACTTATCGCC ACTGGCAGCA GCCACTGGTA 3600
   ACAGGATTAG CAGAGCGAGG TATGTAGGCG GTGCTACAGA GTTCTTGAAG TGGTGGCCTA 3660
   ACTACGGCTA CACTAGAAGG ACAGTATTTG GTATCTGCGC TCTGCTGAAG CCAGTTACCT 3720
   TCGGAAAAAG AGTTGGTAGC TCTTGATCCG GCAAACAAAC CACCGCTGGT AGCGGTGGTT 3780
   TTTTTGTTTG CAAGCAGCAG ATTACGCGCA GAAAAAAAGG ATCTCAAGAA GATCCTTTGA 3840
   TCTTTTCTAC GGGGTCTGAC GCTCAGTGGA ACGAAAACTC ACGTTAAGGG ATTTTGGTCA 3900
   TGAGATTATC AAAAAGGATC TTCACCTAGA TCCTTTTAAA TTAAAAATGA AGTTTTAAAT 3960
   CAATCTAAAG TATATATGAG TAAACTTGGT CTGACAGTTA CCAATGCTTA ATCAGTGAGG 4020
   CACCTATCTC AGCGATCTGT CTATTTCGTT CATCCATAGT TGCCTGACTC CCCGTCGTGT 4080
   AGATAACTAC GATACGGGAG GGCTTACCAT CTGGCCCCAG TGCTGCAATG ATACCGCGAG 4140
   ACCCACGCTC ACCGGCTCCA GATTTATCAG CAATAAACCA GCCAGCCGGA AGGGCCGAGC 4200
   GCAGAAGTGG TCCTGCAACT TTATCCGCCT CCATCCAGTC TATTAATTGT TGCCGGGAAG 4260
   CTAGAGTAAG TAGTTCGCCA GTTAATAGTT TGCGCAACGT TGTTGCCATT GCTACAGGCA 4320
   TCGTGGTGTC ACGCTCGTCG TTTGGTATGG CTTCATTCAG CTCCGGTTCC CAACGATCAA 4380
   GGCGAGTTAC ATGATCCCCC ATGTTGTGCA AAAAAGCGGT TAGCTCCTTC GGTCCTCCGA 4440
   TCGTTGTCAG AAGTAAGTTG GCCGCAGTGT TATCACTCAT GGTTATGGCA GCACTGCATA 4500
   ATTCTCTTAC TGTCATGCCA TCCGTAAGAT GCTTTTCTGT GACTGGTGAG TACTCAACCA 4560
   AGTCATTCTG AGAATAGTGT ATGCGGCGAC CGAGTTGCTC TTGCCCGGCG TCAATACGGG 4620
   ATAATACCGC GCCACATAGC AGAACTTTAA AAGTGCTCAT CATTGGAAAA CGTTCTTCGG 4680
   GGCGAAAACT CTCAAGGATC TTACCGCTGT TGAGATCCAG TTCGATGTAA CCCACTCGTG 4740
   CACCCAACTG ATCTTCAGCA TCTTTTACTT TCACCAGCGT TTCTGGGTGA GCAAAAACAG 4800
   GAAGGCAAAA TGCCGCAAAA AAGGGAATAA GGGCGACACG GAAATGTTGA ATACTCATAC 4860
   TCTTCCTTTT TCAATATTAT TGAAGCATTT ATCAGGGTTA TTGTCTCATG AGCGGATACA 4920
   TATTTGAATG TATTTAGAAA AATAAACAAA TAGGGGTTCC GCGCACATTT CCCCGAAAAG 4980
   TGCCACCTGA CGTCTAAGAA ACCATTATTA TCATGACATT AACCTATAAA AATAGGCGTA 5040
   TCACGAGGCC CTTTCGTCTC GCGCGTTTCG GTGATGACGG TGAAAACCTC TGACACATGC 5100
   AGCTCCCGGA GACGGTCACA GCTTGTCTGT AAGCGGATGC CGGGAGCAGA CAAGCCCGTC 5160
   AGGGCGCGTC AGCGGGTGTT GGCGGGTGTC GGGGCTGGCT TAACTATGCG GCATCAGAGC 5220
   AGATTGTACT GAGAGTGCAC CATATGCGGT GTGAAATACC GCACAGATGC GTAAGGAGAA 5280
   AATACCGCAT CAGG                                                   5294
(2) INFORMATION ZU SEQ ID NO: 3:
   (i) SEQUENZKENNZEICHEN:
      (A) LÄNGE: 5292 Basenpaare
      (B) ART: Nucleotid
      (C) STRANGFORM: Doppelstrang
      (D) TOPOLOGIE: ringförmig
   (ii) ART DES MOLEKÜLS: Genom-DNA
   (xi) SEQUENZBESCHREIBUNG: SEQ ID NO: 3:
   CGATTAGTCC AATTTGTTAA AGACAGGATA TCAGGTGGTC CAGGCTCTAG TTTTGACTCA   60
   ACAATATCAC CAGCTGAAGC CTATAGAGTA CGAGCCATAG ATAGAATAAA AGATTTTATT  120
   TAGTCTCCAG AAAAAGGGGG GAATGAAAGA CCCCACCTGT AGGTTTGGCA AGCTAGCTTA  180
   AGTAACGCCA TTTTGCAAGG CATGGAAAAT ACATAACTGA GAATAGAGAA GTTCAGATCA  240
   AGGTTAGGAA CAGAGAGACA GCAGAATATG GGCCAAACAG GATATCTGTG GTAAGCAGTT 300
   CCTGCCCCGC TCAGGGCCAA GAACAGATGG TCCCCAGATG CGGTCCCGCC CTCAGCAGTT 360
   TCTAGAGAAC CATCAGATGT TTCCAGGGTG CCCCAAGGAC CTGAAAATGA CCCTGTGCCT 420
   TATTTGAACT AACCAATCAG TTCGCTTCTC GCTTCTGTTC GCGCGCTTCT GCTCCCCGAG 480
   CTCAATAAAA GAGCCCACAA CCCCTCACTC GGCGCGCCAG TCCTCCGATT GACTGCGTCG 540
   CCCGGGTACC GTATTCCCAA TAAAGCCTCT TGCTGTTTGC ATCCGAATCG TGGTCTCGCT 600
   GTTCCTTGGG AGGGTCTCCT CTGAGTGATT GACTACCCAC GACGGGGGTC TTTCATTTGG 660
   GGGCTCGTCC GGGATTTGGA GACCCCTGCC CAGGGACCAC CGACCCACCA CCGGGAGGTA 720
   AGCTGGCCAG CAACTTATCT GTGTCTGTCC GATTGTCTAG TGTCTATGTT TGATGTTATG 780
   CGCCTGCGTC TGTACTAGTT AGCTAACTAG CTCTGTATCT GGCGGACCCG TGGTGGAACT 840
   GACGAGTTCT GAACACCCGG CCGCAACCCT GGGAGACGTC CCAGGGACTT TGGGGGCCGT 900
   TTTTGTGGCC CGACCTGAGG AAGGGAGTCG ATGTGGAATC CGACCCCGTC AGGATATGTG 960
   GTTCTGGTAG GAGACGAGAA CCTAAAACAG TTCCCGCCTC CGTCTGAATT TTTGCTTTCG 1020
   GTTTGGAACC GAAGCCGCGC GTCTTGTCTG CTGCAGCATC GTTCTGTGTT GTCTCTGTCT 1080
   GACTGTGTTT CTGTATTTGT CTGAAAATTA GGGCCAGACT GTTACCACTC CCTTAAGTTT 1140
   GACCTTAGAT CACTGGAAAG ATGTCGAGCG GATCGCTCAC AACCAGTCGG TAGATGTCAA 1200
   GAAGAGACGA TGGGTTACCT TCTGCTCTGC AGAATGGCCA ACCTTTAACG TCGGATGGCC 1260
   GCGAGACGGC ACCTTTAACC GAGACCTCAT CACCCAGGTT AAGATCAAGG TCTTTTCACC 1320
   TGGCCCGCAT GGACACCCAG ACCAGGTCCC CTACATCGTG ACCTGGGAAG CCTTGGCTTT 1380
   TGACCCCCCT CCCTGGGTCA AGCCCTTTGT ACACCCTAAG CCTCCGCCTC CTCTTCCTCC 1440
   ATCCGCCCCG TCTCTCCCCC TTGAACCTCC TCTTTCGACC CCGCCTCGAT CCTCCCTTTA 1500
   TCCAGCCCTC ACTCCTTCTC TAGGCGGCTC CACCGCGGTG GCGGCCGCTC TAGAACTAGT 1560
   GGATCCAAGC TTATCGATAG GCCTAGGCCT ATCGATAGGC CTAGGCCTAT CGATAGGCCT 1620
   AACACGAGCC ATAGATAGAA TAAAAGATTT TATTTAGTCT CCAGAAAAAG GGGGGAATGA 1680
   AAGACCCCAC CTGTAGGTTT GGCAAGCTAG AGTCGCTTAG CCTGATAGCC GCAGTAACGC 1740
   CATTTTGCAA GGCATGGAAA AATACCAAAC CAAGAATAGG GAAGTTCAGA TCAAGGGCGG 1800
   GTACATGAAA ATAGCTAACG TTGGGCCAAA CAGGATATCT GCGGTGAGCA GTTTCGGCCC 1860
   CGGCCCGGGG CAAGAACAGA TGGTCACCGC AGTTTCGGCC CCGGCCCGAG GCCAAGAACA 1920
   GATGGTCCCC AGATATGGCC CAACCCTCAG CAGTTTCTTA AGACCCATCA GATGTTTCCA 1980
   GGCTCCCCCA AGGACCTGAA ATGACCCTGC GCCTTATTTG AATTAACCAA TCAGCCTGCT 2040
   TCTCGCTTCT GTTCGCGCGC TTCTGCTTCC CGAGCTCTAT AAAAGAGCTC ACAACCCCTC 2100
   ACTCGGCGCG CCAGTCCTCC GATTGACTGA GTCGCCCGGG TACCCGTGTT CTCAATAAAC 2160
   CCTCTTGCAG TTGCATCCGA CTCGTGGTCT CGCTGTTCCT TGGGAGGGTC TCCTCTGAGT 2220
   GATTGACTAC CCGTCAGCGG GGGTCTTTCA GTTTCTCCCA CCTACACAGG TCTCACTAAC 2280
   ATTCCTGATG TGCCGCAGGG ACTCCGTCAG CCCGGTTTGT GTTTATAATA AAATGCAAGA 2340
   ACAGTGTTCC CTTCAAGCCA GACTACATCC TGACTCTCGG CTTTATAAAA GAATGTTGAA 2400
   GGGCTCTGTG GACTATCTGC CACACGACTT TTAAGATTTT TATGCCTCCT GGATGAGGGA 2460
   TTTAGTCAAT CTATCCTCGT CTATTTTGCT GGCTTCTCCG TATTTTAAAT TTCTAGTTTG 2520
   CACTCCCTTC CTGAGAGCAC GGCGATTGCA GAGTAGTTAA TACTCTGAGG GCAGGCTTCT 2580
   GTGAAAAGGT TGCCTGGGCT CAGTGTGAGA TTTTGCCATA AAAAGGGGTC CTGCCCCTGT 2640
   GTACAGACAG ATCGGAATCT AGAGTGCATA CTCAGAGTCC CCGCGGTTCC GGGGCTCTGA 2700
   TCTCAGGGCA TCTTTGCCTA GAGATCCTCT ACGCCGGACG CATCGTGGCC GGCATCACCG 2760
   GCGCCACAGG TGCGGTTGCT GGCGCCTATA TCGCCGACAT CACCGATGGG GAAGATCGGG 2820
   CTCGCCACTT CGGGCTCATG AGCGCTTGTT TCGGCGTGGG TATGGTGGCA GGCCCCGTGG 2880
   CCGGGGGACT GTTGGGCGCC ATCTCCTTGC ATGCACCATT CCTTGCGGCG GCGGTGCTCA 2940
   ACGGCCTCAA CCTACTACTG GGCTGCTTCC TAATGCAGGA GTCGCATAAG GGAGAGCGTC 3000
   CTGCATTAAT GAATCGGCCA ACGCGCGGGG AGAGGCGGTT TGCGTATTGG GCGCTCTTCC 3060
   GCTTCCTCGC TCACTGACTC GCTGCGCTCG GTCGTTCGGC TGCGGCGAGC GGTATCAGCT 3120
   CACTCAAAGG CGGTAATACG GTTATCCACA GAATCAGGGG ATAACGCAGG AAAGAACATG 3180
   TGAGCAAAAG GCCAGCAAAA GGCCAGGAAC CGTAAAAAGG CCGCGTTGCT GGCGTTTTTC 3240
   CATAGGCTCC GCCCCCCTGA CGAGCATCAC AAAAATCGAC GCTCAAGTCA GAGGTGGCGA 3300
   AACCCGACAG GACTATAAAG ATACCAGGCG TTTCCCCCTG GAAGCTCCCT CGTGCGCTCT 3360
   CCTGTTCCGA CCCTGCCGCT TACCGGATAC CTGTCCGCCT TTCTCCCTTC GGGAAGCGTG 3420
   GCGCTTTCTC AATGCTCACG CTGTAGGTAT CTCAGTTCGG TGTAGGTCGT TCGCTCCAAG 3480
   CTGGGCTGTG TGCACGAACC CCCCGTTCAG CCCGACCGCT GCGCCTTATC CGGTAACTAT 3540
   CGTCTTGAGT CCAACCCGGT AAGACACGAC TTATCGCCAC TGGCAGCAGC CACTGGTAAC 3600
   AGGATTAGCA GAGCGAGGTA TGTAGGCGGT GCTACAGAGT TCTTGAAGTG GTGGCCTAAC 3660
   TACGGCTACA CTAGAAGGAC AGTATTTGGT ATCTGCGCTC TGCTGAAGCC AGTTACCTTC 3720
   GGAAAAAGAG TTGGTAGCTC TTGATCCGGC AAACAAACCA CCGCTGGTAG CGGTGGTTTT 3780
   TTTGTTTGCA AGCAGCAGAT TACGCGCAGA AAAAAAGGAT CTCAAGAAGA TCCTTTGATC 3840
   TTTTCTACGG GGTCTGACGC TCAGTGGAAC GAAAACTCAC GTTAAGGGAT TTTGGTCATG 3900
   AGATTATCAA AAAGGATCTT CACCTAGATC CTTTTAAATT AAAAATGAAG TTTTAAATCA 3960
   ATCTAAAGTA TATATGAGTA AACTTGGTCT GACAGTTACC AATGCTTAAT CAGTGAGGCA 4020
   CCTATCTCAG CGATCTGTCT ATTTCGTTCA TCCATAGTTG CCTGACTCCC CGTCGTGTAG 4080
   ATAACTACGA TACGGGAGGG CTTACCATCT GGCCCCAGTG CTGCAATGAT ACCGCGAGAC 4140
   CCACGCTCAC CGGCTCCAGA TTTATCAGCA ATAAACCAGC CAGCCGGAAG GGCCGAGCGC 4200
   AGAAGTGGTC CTGCAACTTT ATCCGCCTCC ATCCAGTCTA TTAATTGTTG CCGGGAAGCT 4260
   AGAGTAAGTA GTTCGCCAGT TAATAGTTTG CGCAACGTTG TTGCCATTGC TACAGGCATC 4320
   GTGGTGTCAC GCTCGTCGTT TGGTATGGCT TCATTCAGCT CCGGTTCCCA ACGATCAAGG 4380
   CGAGTTACAT GATCCCCCAT GTTGTGCAAA AAAGCGGTTA GCTCCTTCGG TCCTCCGATC 4440
   GTTGTCAGAA GTAAGTTGGC CGCAGTGTTA TCACTCATGG TTATGGCAGC ACTGCATAAT 4500
   TCTCTTACTG TCATGCCATC CGTAAGATGC TTTTCTGTGA CTGGTGAGTA CTCAACCAAG 4560
   TCATTCTGAG AATAGTGTAT GCGGCGACCG AGTTGCTCTT GCCCGGCGTC AATACGGGAT 4620
   AATACCGCGC CACATAGCAG AACTTTAAAA GTGCTCATCA TTGGAAAACG TTCTTCGGGG 4680
   CGAAAACTCT CAAGGATCTT ACCGCTGTTG AGATCCAGTT CGATGTAACC CACTCGTGCA 4740
   CCCAACTGAT CTTCAGCATC TTTTACTTTC ACCAGCGTTT CTGGGTGAGC AAAAACAGGA 4800
   AGGCAAAATG CCGCAAAAAA GGGAATAAGG GCGACACGGA AATGTTGAAT ACTCATACTC 4860
   TTCCTTTTTC AATATTATTG AAGCATTTAT CAGGGTTATT GTCTCATGAG CGGATACATA 4920
   TTTGAATGTA TTTAGAAAAA TAAACAAATA GGGGTTCCGC GCACATTTCC CCGAAAAGTG 4980
   CCACCTGACG TCTAAGAAAC CATTATTATC ATGACATTAA CCTATAAAAA TAGGCGTATC 5040
   ACGAGGCCCT TTCGTCTCGC GCGTTTCGGT GATGACGGTG AAAACCTCTG ACACATGCAG 5100
   CTCCCGGAGA CGGTCACAGC TTGTCTGTAA GCGGATGCCG GGAGCAGACA AGCCCGTCAG 5160
   GGCGCGTCAG CGGGTGTTGG CGGGTGTCGG GGCTGGCTTA ACTATGCGGC ATCAGAGCAG 5220
   ATTGTACTGA GAGTGCACCA TATGCGGTGT GAAATACCGC ACAGATGCGT AAGGAGAAAA 5280
   TACCGCATCA GG 5292
(2) INFORMATION ZU SEQ ID NO: 4:
   (i) SEQUENZKENNZEICHEN:
      (A) LÄNGE: 5364 Basenpaare
      (B) ART: Nucleotid
      (C) STRANGFORM: Doppelstrang
      (D) TOPOLOGIE: ringförmig
   (ii) ART DES MOLEKÜLS: Genom-DNA
   (xi) SEQUENZBESCHREIBUNG: SEQ ID NO: 4:
   CGATTAGTCC AATTTGTTAA AGACAGGATA TCAGGTGGTC CAGGCTCTAG TTTTGACTCA 60
   ACAATATCAC CAGCTGAAGC CTATAGAGTA CGAGCCATAG ATAGAATAAA AGATTTTATT 120
   TAGTCTCCAG AAAAAGGGGG GAATGAAAGA CCCCACCTGT AGGTTTGGCA AGCTAGCTTA 180
   AGTAACGCCA TTTTGCAAGG CATGGAAAAT ACATAACTGA GAATAGAGAA GTTCAGATCA 240
   AGGTTAGGAA CAGAGAGACA GCAGAATATG GGCCAAACAG GATATCTGTG GTAAGCAGTT 300
   CCTGCCCCGC TCAGGGCCAA GAACAGATGG TCCCCAGATG CGGTCCCGCC CTCAGCAGTT 360
   TCTAGAGAAC CATCAGATGT TTCCAGGGTG CCCCAAGGAC CTGAAAATGA CCCTGTGCCT 420
   TATTTGAACT AACCAATCAG TTCGCTTCTC GCTTCTGTTC GCGCGCTTCT GCTCCCCGAG 480
   CTCAATAAAA GAGCCCACAA CCCCTCACTC GGCGCGCCAG TCCTCCGATT GACTGCGTCG 540
   CCCGGGTACC CGTATTCCCA ATAAAGCCTC TTGCTGTTTG CATCCGAATC GTGGACTCGC 600
   TGATCCTTGG GAGGGTCTCC TCAGATTGAT TGACTGCCCA CCTCGGGGGT CTTTCATTTG 660
   GAGGTTCCAC CGAGATTTGG AGACCCCAGC CCAGGGACCA CCGACCCCCC CGCCGGGAGG 720
   TAAGCTGGCC AGCGGTCGTT TCGTGTCTGT CTCTGTCTTT GTGCGTGTTT GTGCCGGCAT 780
   CTAATGTTTG CGCCTGCGTC TGTACTAGTT GGCTAACTAG ATCTGTATCT GGCGGTCCCG 840
   CGGAAGAACT GACGAGTTCG TATTCCCGGC CGCAGCCCCT AGGAGACGTC CCAGCGGCCT 900
   CGGGGGCCCG TTTTGTGGCC CGTTCTGTGT CGTTAACCAC CCGAGTCGGA CTTTTTGGAG 960
   CTCCGCCACT GTCCGAGGGG TACGTGGCTT TGTTGGGGGA CGAGAGACAG AGACACTTCC 1020
   CGCCCCCGTC TGAATTTTTG CTTTCGGTTT TACGCCGAAA CCGCGCCGCG CGTCTTGTCT 1080
   GCTGCAGCAT CGTTCTGTGT TGTCTCTGTC TGACTGTGTT TCTGTATTTG TCTGAAAATT 1140
   AGGGCCAGAC TGTTACCACT CCCTTAAGTT TGACCTTAGA TCACTGGAAA GATGTCGAGC 1200
   GGATCGCTCA CAACCAGTCG GTAGATGTCA AGAAGAGACG ATGGGTTACC TTCTGCTCTG 1260
   CAGAATGGCC AACCTTTAAC GTCGGATGGC CGCGAGACGG CACCTTTAAC CGAGACCTCA 1320
   TCACCCAGGT TAAGATCAAG GTCTTTTCAC CTGGCCCGCA TGGACACCCA GACCAGGTCC 1380
   CCTACATCGT GACCTGGGAA GCCTTGGCTT TTGACCCCCC TCCCTGGGTC AAGCCCTTTG 1440
   TACACCCTAA GCCTCCGCCT CCTCTTCCTC CATCCGCCCC GTCTCTCCCC CTTGAACCTC 1500
   CTCTTTCGAC CCCGCCTCGA TCCTCCCTTT ATCCAGCCCT CACTCCTTCT CTAGGCGGCT 1560
   CCACCGCGGT GGCGGCCGCT CTAGAACTAG TGGATCCAAG CTTATCGATA GGCCTAGGCC 1620
   TATCGATAGG CCTAGGCCTA TCGATAGGCC TAACACGAGC CATAGATAGA ATAAAAGATT 1680
   TTATTTAGTC TCCAGAAAAA GGGGGGAATG AAAGACCCCA CCTGTAGGTT TGGCAAGCTA 1740
   GAGTCGCTTA GCCTGATAGC CGCAGTAACG CCATTTTGCA AGGCATGGAA AAATACCAAA 1800
   CCAAGAATAG GGAAGTTCAG ATCAAGGGCG GGTACATGAA AATAGCTAAC GTTGGGCCAA 1860
   ACAGGATATC TGCGGTGAGC AGTTTCGGCC CCGGCCCGGG GCAAGAACAG ATGGTCACCG 1920
   CAGTTTCGGC CCCGGCCCGG GCCAAGAACA GATGGTCACC GCAGTTTCGG CCCCGGCCCG 1980
   GGGCCAAGAA CAGATGGTCC CCAGATATGG CCCAACCCTC AGCAGTTTCT TAAGACCCAT 2040
   CAGATGTTTC CAGGCTCCCC CAAGGACCTG AAATGACCCT GCGCCTTATT TGAATTAACC 2100
   AATCAGCCTG CTTCTCGCTT CTGTTCGCGC GCTTCTGCTT CCCGAGCTCT ATAAAAGAGC 2160
   TCACAACCCC TCACTCGGCG CGCCAGTCCT CCGATTGACT GAGTCGCCCG GGTACCCGTG 2220
   TTCTCAATAA ACCCTCTTGC AGTTGCATCC GACTCGTGGT CTCGCTGTTC CTTGGGAGGG 2280
   TCTCCTCTGA GTGATTGACT ACCCGTCAGC GGGGGTCTTT CAGTTTCTCC CACCTACACA 2340
   GGTCTCACTA ACATTCCTGA TGTGCCGCAG GGACTCCGTC AGCCCGGTTT GTGTTTATAA 2400
   TAAAATGCAA GAACAGTGTT CCCTTCAAGC CAGACTACAT CCTGACTCTC GGCTTTATAA 2460
   AAGAATGTTG AAGGGCTCTG TGGACTATCT GCCACACGAC TTTTAAGATT TTTATGCCTC 2520
   CTGGATGAGG GATTTAGTCA ATCTATCCTC GTCTATTTTG CTGGCTTCTC CGTATTTTAA 2580
   ATTTCTAGTT TGCACTCCCT TCCTGAGAGC ACGGCGATTG CAGAGTAGTT AATACTCTGA 2640
   GGGCAGGCTT CTGTGAAAAG GTTGCCTGGG CTCAGTGTGA GATTTTGCCA TAAAAAGGGG 2700
   TCCTGCCCCT GTGTACAGAC AGATCGGAAT CTAGAGTGCA TACTCAGAGT CCCCGCGGTT 2760
   CCGGGGCTCT GATCTCAGGG CATCTTTGCC TAGAGATCCT CTACGCCGGA CGCATCGTGG 2820
   CCGGCATCAC CGGCGCCACA GGTGCGGTTG CTGGCGCCTA TATCGCCGAC ATCACCGATG 2880
   GGGAAGATCG GGCTCGCCAC TTCGGGCTCA TGAGCGCTTG TTTCGGCGTG GGTATGGTGG 2940
   CAGGCCCCGT GGCCGGGGGA CTGTTGGGCG CCATCTCCTT GCATGCACCA TTCCTTGCGG 3000
   CGGCGGTGCT CAACGGCCTC AACCTACTAC TGGGCTGCTT CCTAATGCAG GAGTCGCATA 3060
   AGGGAGAGCG TCCTGCATTA ATGAATCGGC CAACGCGCGG GGAGAGGCGG TTTGCGTATT 3120
   GGGCGCTCTT CCGCTTCCTC GCTCACTGAC TCGCTGCGCT CGGTCGTTCG GCTGCGGCGA 3180
   GCGGTATCAG CTCACTCAAA GGCGGTAATA CGGTTATCCA CAGAATCAGG GGATAACGCA 3240
   GGAAAGAACA TGTGAGCAAA AGGCCAGCAA AAGGCCAGGA ACCGTAAAAA GGCCGCGTTG 3300
   CTGGCGTTTT TCCATAGGCT CCGCCCCCCT GACGAGCATC ACAAAAATCG ACGCTCAAGT 3360
   CAGAGGTGGC GAAACCCGAC AGGACTATAA AGATACCAGG CGTTTCCCCC TGGAAGCTCC 3420
   CTCGTGCGCT CTCCTGTTCC GACCCTGCCG CTTACCGGAT ACCTGTCCGC CTTTCTCCCT 3480
   TCGGGAAGCG TGGCGCTTTC TCAATGCTCA CGCTGTAGGT ATCTCAGTTC GGTGTAGGTC 3540
   GTTCGCTCCA AGCTGGGCTG TGTGCACGAA CCCCCCGTTC AGCCCGACCG CTGCGCCTTA 3600
   TCCGGTAACT ATCGTCTTGA GTCCAACCCG GTAAGACACG ACTTATCGCC ACTGGCAGCA 3660
   GCCACTGGTA ACAGGATTAG CAGAGCGAGG TATGTAGGCG GTGCTACAGA GTTCTTGAAG 3720
   TGGTGGCCTA ACTACGGCTA CACTAGAAGG ACAGTATTTG GTATCTGCGC TCTGCTGAAG 3780
   CCAGTTACCT TCGGAAAAAG AGTTGGTAGC TCTTGATCCG GCAAACAAAC CACCGCTGGT 3840
   AGCGGTGGTT TTTTTGTTTG CAAGCAGCAG ATTACGCGCA GAAAAAAAGG ATCTCAAGAA 3900
   GATCCTTTGA TCTTTTCTAC GGGGTCTGAC GCTCAGTGGA ACGAAAACTC ACGTTAAGGG 3960
   ATTTTGGTCA TGAGATTATC AAAAAGGATC TTCACCTAGA TCCTTTTAAA TTAAAAATGA 4020
   AGTTTTAAAT CAATCTAAAG TATATATGAG TAAACTTGGT CTGACAGTTA CCAATGCTTA 4080
   ATCAGTGAGG CACCTATCTC AGCGATCTGT CTATTTCGTT CATCCATAGT TGCCTGACTC 4140
   CCCGTCGTGT AGATAACTAC GATACGGGAG GGCTTACCAT CTGGCCCCAG TGCTGCAATG 4200
   ATACCGCGAG ACCCACGCTC ACCGGCTCCA GATTTATCAG CAATAAACCA GCCAGCCGGA 4260
   AGGGCCGAGC GCAGAAGTGG TCCTGCAACT TTATCCGCCT CCATCCAGTC TATTAATTGT 4320
   TGCCGGGAAG CTAGAGTAAG TAGTTCGCCA GTTAATAGTT TGCGCAACGT TGTTGCCATT 4380
   GCTACAGGCA TCGTGGTGTC ACGCTCGTCG TTTGGTATGG CTTCATTCAG CTCCGGTTCC 4440
   CAACGATCAA GGCGAGTTAC ATGATCCCCC ATGTTGTGCA AAAAAGCGGT TAGCTCCTTC 4500
   GGTCCTCCGA TCGTTGTCAG AAGTAAGTTG GCCGCAGTGT TATCACTCAT GGTTATGGCA 4560
   GCACTGCATA ATTCTCTTAC TGTCATGCCA TCCGTAAGAT GCTTTTCTGT GACTGGTGAG 4620
   TACTCAACCA AGTCATTCTG AGAATAGTGT ATGCGGCGAC CGAGTTGCTC TTGCCCGGCG 4680
   TCAATACGGG ATAATACCGC GCCACATAGC AGAACTTTAA AAGTGCTCAT CATTGGAAAA 4740
   CGTTCTTCGG GGCGAAAACT CTCAAGGATC TTACCGCTGT TGAGATCCAG TTCGATGTAA 4800
   CCCACTCGTG CACCCAACTG ATCTTCAGCA TCTTTTACTT TCACCAGCGT TTCTGGGTGA 4860
   GCAAAAACAG GAAGGCAAAA TGCCGCAAAA AAGGGAATAA GGGCGACACG GAAATGTTGA 4920
   ATACTCATAC TCTTCCTTTT TCAATATTAT TGAAGCATTT ATCAGGGTTA TTGTCTCATG 4980
   AGCGGATACA TATTTGAATG TATTTAGAAA AATAAACAAA TAGGGGTTCC GCGCACATTT 5040
   CCCCGAAAAG TGCCACCTGA CGTCTAAGAA ACCATTATTA TCATGACATT AACCTATAAA 5100
   AATAGGCGTA TCACGAGGCC CTTTCGTCTC GCGCGTTTCG GTGATGACGG TGAAAACCTC 5160
   TGACACATGC AGCTCCCGGA GACGGTCACA GCTTGTCTGT AAGCGGATGC CGGGAGCAGA 5220
   CAAGCCCGTC AGGGCGCGTC AGCGGGTGTT GGCGGGTGTC GGGGCTGGCT TAACTATGCG 5280
   GCATCAGAGC AGATTGTACT GAGAGTGCAC CATATGCGGT GTGAAATACC GCACAGATGC 5340
   GTAAGGAGAA AATACCGCAT CAGG 5364
(2) ANGABEN ZU SEQ ID NO: 5: (Nur zur technischen Information)
   (i) SEQUENZKENNZEICHEN:
      (A) LÄNGE: 6505 Basenpaare
      (B) ART: Nucleotid
      (C) STRANGFORM: Doppelstrang
      (D) TOPOLOGIE: ringförmig
   (ii) ART DES MOLEKÜLS: Genom-DNA
   (xi) SEQUENZBESCHREIBUNG: SEQ ID NO: 5:
   TCGAGGGGGG GCCCGGTACC GATTAGTCCA ATTTGTTAAA GACAGGATAT CAGGTGGTCC 60
   AGGCTCTAGT TTTGACTCAA CAATATCACC AGCTGAAGCC TATAGAGTAC GAGCCATAGA 120
   TAGAATAAAA GATTTTATTT AGTCTCCAGA AAAAGGGGGG AATGAAAGAC CCCACCTGTA 180
   GGTTTGGCAA GCTAGCTTAA GTAACGCCAT TTTGCAAGGC ATGGAAAATA CATAACTGAG 240
   AATAGAGAAG TTCAGATCAA GGTTAGGAAC AGAGAGACAG CAGAATATGG GCCAAACAGG 300
   ATATCTGTGG TAAGCAGTTC CTGCCCCGCT CAGGGCCAAG AACAGATGGT CCCCAGATGC 360
   GGTCCCGCCC TCAGCAGTTT CTAGAGAACC ATCAGATGTT TCCAGGGTGC CCCAAGGACC 420
   TGAAAATGAC CCTGTGCCTT ATTTGAACTA ACCAATCAGT TCGCTTCTCG CTTCTGTTCG 480
   CGCGCTTCTG CTCCCCGAGC TCAATAAAAG AGCCCACAAC CCCTCACTCG GCGCGCCAGT 540
   CCTCCGATTG ACTGCGTCGC CCGGGTACCC GTATTCCCAA TAAAGCCTCT TGCTGTTTGC 600
   ATCCGAATCG TGGACTCGCT GATCCTTGGG AGGGTCTCCT CAGATTGATT GACTGCCCAC 660
   CTCGGGGGTC TTTCATTTGG AGGTTCCACC GAGATTTGGA GACCCCAGCC CAGGGACCAC 720
   CGACCCCCCC GCCGGGAGGT AAGCTGGCCA GCGGTCGTTT CGTGTCTGTC TCTGTCTTTG 780
   TGCGTGTTTG TGCCGGCATC TAATGTTTGC GCCTGCGTCT GTACTAGTTG GCTAACTAGA 840
   TCTGTATCTG GCGGTCCCGC GGAAGAACTG ACGAGTTCGT ATTCCCGGCC GCAGCCCCTA 900
   GGAGACGTCC CAGCGGCCTC GGGGGCCCGT TTTGTGGCCC GTTCTGTGTC GTTAACCACC 960
   CGAGTCGGAC TTTTTGGAGC TCCGCCACTG TCCGAGGGGT ACGTGGCTTT GTTGGGGGAC 1020
   GAGAGACAGA GACACTTCCC GCCCCCGTCT GAATTTTTGC TTTCGGTTTT ACGCCGAAAC 1080
   CGCGCCGCGC GTCTTGTCTG CTGCAGCATC GTTCTGTGTT GTCTCTGTCT GACTGTGTTT 1140
   CTGTATTTGT CTGAAAATTA GGGCCAGACT GTTACCACTC CCTTAAGTTT GACCTTAGAT 1200
   CACTGGAAAG ATGTCGAGCG GATCGCTCAC AACCAGTCGG TAGATGTCAA GAAGAGACGA 1260
   TGGGTTACCT TCTGCTCTGC AGAATGGCCA ACCTTTAACG TCGGATGGCC GCGAGACGGC 1320
   ACCTTTAACC GAGACCTCAT CACCCAGGTT AAGATCAAGG TCTTTTCACC TGGCCCGCAT 1380
   GGACACCCAG ACCAGGTCCC CTACATCGTG ACCTGGGAAG CCTTGGCTTT TGACCCCCCT 1440
   CCCTGGGTCA AGCCCTTTGT ACACCCTAAG CCTCCGCCTC CTCTTCCTCC ATCCGCCCCG 1500
   TCTCTCCCCC TTGAACCTCC TCTTTCGACC CCGCCTCGAT CCTCCCTTTA TCCAGCCCTC 1560
   ACTCCTTCTC TAGGCGGCTC CACCGCGGTG GCGGCCGATC CCCCGGGCTG CAGGAATTCG 1620
   ATATCAAGCT TATCGATACC GTCGACTCTA GAGGATCCCG CCGCGGCGGG TACCGAGCTC 1680
   ATTCGAGTAG CGGCTCTTCC AAGCTCAAAG AAGCAGAGGC CGCTGTTCGT TTCCTTTAGG 1740
   TCTTTCCACT AAAGTCGGAG TATCTTCTTC CAAGATTTCA CGTCTTGGTG GCCGTTCCAA 1800
   GGAGCGCGAG GTCGGGATGG ATCTTGAAGG GGACCGCAAT GGAGGAGCAA AGAAGAAGAA 1860
   CTTTTTTAAA CTGAACAATA AAAGTGAAAA AGATAAGAAG GAAAAGAAAC CAACTGTCAG 1920
   TGTATTTTCA ATGTTTCGCT ATTCAAATTG GCTTGACAAG TTGTATATGG TGGTGGGAAC 1980
   TTTGGCTGCC ATCATCCATG GGGCTGGACT TCCTCTCATG ATGCTGGTGT TTGGAGAAAT 2040
   GACAGATATC TTTGCAAATG CAGGAAATTT AGAAGATCTG ATGTCAAACA TCACTAATAG 2100
   AAGTGATATC AATGATACAG GGTTCTTCAT GAATCTGGAG GAAGACATGA CCAGGTATGC 2160
   CTATTATTAC AGTGGAATTG GTGCTGGGGT GCTGGTTGCT GCTTACATTC AGGTTTCATT 2220
   TTGGTGCCTG GCAGCTGGAA GACAAATACA CAAAATTAGA AAACAGTTTT TTCATGCTAT 2280
   AATGCGACAG GAGATAGGCT GGTTTGATGT GCACGATGTT GGGGAGCTTA ACACCCGACT 2340
   TACAGATGAT GTCTCTAAGA TTAATGAAGT TATTGGTGAC AAAATTGGAA TGTTCTTTCA 2400
   GTCAATGGCA ACATTTTTCA CTGGGTTTAT AGTAGGATTT ACACGTGGTT GGAAGCTAAC 2460
   CCTTGTGATT TTGGCCATCA GTCCTGTTCT TGGACTGTCA GCTGCTGTCT GGGCAAAGAT 2520
   ACTATCTTCA TTTACTGATA AAGAACTCTT AGCGTATGCA AAAGCTGGAG CAGTAGCTGA 2580
   AGAGGTCTTG GCAGCAATTA GAACTGTGAT TGCATTTGGA GGACAAAAGA AAGAACTTGA 2640
   AAGGTACAAC AAAAATTTAG AAGAAGCTAA AAGAATTGGG ATAAAGAAAG CTATTACAGC 2700
   CAATATTTCT ATAGGTGCTG CTTTCCTGCT GATCTATGCA TCTTATGCTC TGGCCTTCTG 2760
   GTATGGGACC ACCTTGGTCC TCTCAGGGGA ATATTCTATT GGACAAGTAC TCACTGTATT 2820
   CTTTTCTGTA TTAATTGGGG CTTTTAGTGT TGGACAGGCA TCTCCAAGCA TTGAAGCATT 2880
   TGCAAATGCA AGAGGAGCAG CTTATGAAAT CTTCAAGATA ATTGATAATA AGCCAAGTAT 2940
   TGACAGCTAT TCGAAGAGTG GGCACAAACC AGATAATATT AAGGGAAATT TGGAATTCAG 3000
   AAATGTTCAC TTCAGTTACC CATCTCGAAA AGAAGTTAAG ATCTTGAAGG GCCTGAACCT 3060
   GAAGGTGCAG AGTGGGCAGA CGGTGGCCCT GGTTGGAAAC AGTGGCTGTG GGAAGAGCAC 3120
   AACAGTCCAG CTGATGCAGA GGCTCTATGA CCCCACAGAG GGGATGGTCA GTGTTGATGG 3180
   ACAGGATATT AGGACCATAA ATGTAAGGTT TCTACGGGAA ATCATTGGTG TGGTGAGTCA 3240
   GGAACCTGTA TTGTTTGCCA CCACGATAGC TGAAAACATT CGCTATGGCC GTGAAAATGT 3300
   CACCATGGAT GAGATTGAGA AAGCTGTCAA GGAAGCCAAT GCCTATGACT TTATCATGAA 3360
   ACTGCCTCAT AAATTTGACA CCCTGGTTGG AGAGAGAGGG GCCCAGTTGA GTGGTGGGCA 3420
   GAAGCAGAGG ATCGCCATTG CACGTGCCCT GGTTCGCAAC CCCAAGATCC TCCTGCTGGA 3480
   TGAGGCCACG TCAGCCTTGG ACACAGAAAG CGAAGCAGTG GTTCAGGTGG CTCTGGATAA 3540
   GGCCAGAAAA GGTCGGACCA CCATTGTGAT AGCTCATCGT TTGTCTACAG TTCGTAATGC 3600
   TGACGTCATC GCTGGTTTCG ATGATGGAGT CATTGTGGAG AAAGGAAATC ATGATGAACT 3660
   CATGAAAGAG AAAGGCATTT ACTTCAAACT TGTCACAATG CAGACAGCAG GAAATGAAGT 3720
   TGAATTAGAA AATGCAGCTG ATGAATCCAA AAGTGAAATT GATGCCTTGG AAATGTCTTC 3780
   AAATGATTCA AGATCCAGTC TAATAAGAAA AAGATCAACT CGTAGGAGTG TCCGTGGATC 3840
   ACAAGCCCAA GACAGAAAGC TTAGTACCAA AGAGGCTCTG GATGAAAGTA TACCTCCAGT 3900
   TTCCTTTTGG AGGATTATGA AGCTAAATTT AACTGAATGG CCTTATTTTG TTGTTGGTGT 3960
   ATTTTGTGCC ATTATAAATG GAGGCCTGCA ACCAGCATTT GCAATAATAT TTTCAAAGAT 4020
   TATAGGGGTT TTTACAAGAA TTGATGATCC TGAAACAAAA CGACAGAATA GTAACTTGTT 4080
   TTCACTATTG TTTCTAGCCC TTGGAATTAT TTCTTTTATT ACATTTTTCC TTCAGGGTTT 4140
   CACATTTGGC AAAGCTGGAG AGATCCTCAC CAAGCGGCTC CGATACATGG TTTTCCGATC 4200
   CATGCTCAGA CAGGATGTGA GTTGGTTTGA TGACCCTAAA AACACCACTG GAGCATTGAC 4260
   TACCAGGCTC GCCAATGATG CTGCTCAAGT TAAAGGGGCT ATAGGTTCCA GGCTTGCTGT 4320
   AATTACCCAG AATATAGCAA ATCTTGGGAC AGGAATAATT ATATCCTTCA TCTATGGTTG 4380
   GCAACTAACA CTGTTACTCT TAGCAATTGT ACCCATCATT GCAATAGCAG GAGTTGTTGA 4440
   AATGAAAATG TTGTCTGGAC AAGCACTGAA AGATAAGAAA GAACTAGAAG GTGCTGGGAA 4500
   GATCGCTACT GAAGCAATAG AAAACTTCCG AACCGTTGTT TCTTTGACTC AGGAGCAGAA 4560
   GTTTGAACAT ATGTATGCTC AGAGTTTGCA GGTACCATAC AGAAACTCTT TGAGGAAAGC 4620
   ACACATCTTT GGAATTACAT TTTCCTTCAC CCAGGCAATG ATGTATTTTT CCTATGCTGG 4680
   ATGTTTCCGG TTTGGAGCCT ACTTGGTGGC ACATAAACTC ATGAGCTTTG AGGATGTTCT 4740
   GTTAGTATTT TCAGCTGTTG TCTTTGGTGC CATGGCCGTG GGGCAAGTCA GTTCATTTGC 4800
   TCCTGACTAT GCCAAAGCCA AAATATCAGC AGCCCACATC ATCATGATCA TTGAAAAAAC 4860
   CCCTTTGATT GACAGCTACA GCACGGAAGG CCTAATGCCG AACACATTGG AAGGAAATGT 4920
   CACATTTGGT GAAGTTGTAT TCAACTATCC CACCCGACCG GACATCCCAG TGCTTCAGGG 4980
   ACTGAGCCTG GAGGTGAAGA AGGGCCAGAC GCTGGCTCTG GTGGGCAGCA GTGGCTGTGG 5040
   GAAGAGCACA GTGGTCCAGC TCCTGGAGCG GTTCTACGAC CCCTTGGCAG GGAAAGTGCT 5100
   GCTTGATGGC AAAGAAATAA AGCGACTGAA TGTTCAGTGG CTCCGAGCAC ACCTGGGCAT 5160
   CGTGTCCCAG GAGCCCATCC TGTTTGACTG CAGCATTGCT GAGAACATTG CCTATGGAGA 5220
   CAACAGCCGG GTGGTGTCAC AGGAAGAGAT CGTGAGGGCA GCAAAGGAGG CCAACATACA 5280
   TGCCTTCATC GAGTCACTGC CTAATAAATA TAGCACTAAA GTAGGAGACA AAGGAACTCA 5340
   GCTCTCTGGT GGCCAGAAAC AACGCATTGC CATAGCTCGT GCCCTTGTTA GACAGCCTCA 5400
   TATTTTGCTT TTGGATGAAG CCACGTCAGC TCTGGATACA GAAAGTGAAA AGGTTGTCCA 5460
   AGAAGCCCTG GACAAAGCCA GAGAAGGCCG CACCTGCATT GTGATTGCTC ACCGCCTGTC 5520
   CACCATCCAG AATGCAGACT TAATAGTGGT GTTTCAGAAT GGCAGAGTCA AGGAGCATGG 5580
   CACGCATCAG CAGCTGCTGG CACAGAAAGG CATCTATTTT TCAATGGTCA GTGTCCAGGC 5640
   TGGAACAAAG CGCCAGTGAA CTCTGACTGT ATGAGATGTT AAATACTTTT TAATGGGATC 5700
   CACTAGTTCT AGAGCGGCCG CCACCGCGGT GGAGCTATCC ACCATCATGG GGCTTCTCAT 5760
   TATACTCCTA CTCCTACTAA TTCTGCTTTT GTGGACCCTG CATTCTTAAT CGATTAGTTC 5820
   AATTTGTTAA AGACAGGATC TCAGTAGTCC AGGCTTTAGT CCTGACTCAA CAATACCACC 5880
   AGCTAAAACC ACTAGAATAC GAGCCACAAT AAATAAAAGA TTTTATTTAG TTTCCAGAAA 5940
   AAGGGGGGAA TGAAAGACCC CACCAAGTTG CTTAGCCTGA TGCCGCTGTA ACGCCATTTT 6000
   GCAAGGCATG GAAAAATACC AAACCAAGAA TAGAGAAGTT CAGATCAAGG GCGGGTACAT 6060
   GAAAATAGCT AACGTTGGGC CAAACAGGAT ATCTGCGGTG AGCAGTTTCG GCCCCGGCCC 6120
   GGGGCAAGAA CAGATGGTCA CCGCAGTTTC GGCCCCGGCC CGAGGCCAAG AACAGATGGT 6180
   CCCCAGATAT GGCCCAACCC TCAGCAGTTT CTTAAGACCC ATCAGATGTT TCCAGGCTCC 6240
   CCCAAGGACC TGAAATGACC CTGCGCCTTA TTTGAATTAA CCAATCAGCC TGCTTCTCGC 6300
   TTCTGTTCGC GCGCTTCTGC TTCCCGAGCT CTATAAAAGA GCTCACAACC CCTCACTCGG 6360
   CGCGCCAGTC CTCCGATTGA CTGAGTCGCC CGGGTACCCG TGTTCCCAAT AAAGCCTCTT 6420
   GCTGATTGCA TCCGAATCGT GGACTCGCTG ATCCTTGGGA GGGTCTCCTC AGATTGATTG 6480
   ACTGCCCACC TGGGGGTCTT TCAGT                                       6505
(2) ANGABEN ZU SEQ ID NO: 6:
   (i) SEQUENZKENNZEICHEN:
      (A) LÄNGE: 9318 Basenpaare
      (B) ART: Nucleotid
      (C) STRANGFORM: Doppelstrang
      (D) TOPOLOGIE: ringförmig
   (ii) ART DES MOLEKÜLS: Genom-DNA
   (xi) SEQUENZBESCHREIBUNG: SEQ ID NO: 6:
   TCGAGGGGGG GCCCGGTACC GATTAGTCCA ATTTGTTAAA GACAGGATAT CAGGTGGTCC   60
   AGGCTCTAGT TTTGACTCAA CAATATCACC AGCTGAAGCC TATAGAGTAC GAGCCATAGA  120
   TAGAATAAAA GATTTTATTT AGTCTCCAGA AAAAGGGGGG AATGAAAGAC CCCACCTGTA  180
   GGTTTGGCAA GCTAGCTTAA GTAACGCCAT TTTGCAAGGC ATGGAAAATA CATAACTGAG  240
   AATAGAGAAG TTCAGATCAA GGTTAGGAAC AGAGAGACAG CAGAATATGG GCCAAACAGG  300
   ATATCTGTGG TAAGCAGTTC CTGCCCCGCT CAGGGCCAAG AACAGATGGT CCCCAGATGC  360
   GGTCCCGCCC TCAGCAGTTT CTAGAGAACC ATCAGATGTT TCCAGGGTGC CCCAAGGACC  420
   TGAAAATGAC CCTGTGCCTT ATTTGAACTA ACCAATCAGT TCGCTTCTCG CTTCTGTTCG  480
   CGCGCTTCTG CTCCCCGAGC TCAATAAAAG AGCCCACAAC CCCTCACTCG GCGCGCCAGT  540
   CCTCCGATTG ACTGCGTCGC CCGGGTACCC GTATTCCCAA TAAAGCCTCT TGCTGTTTGC  600
   ATCCGAATCG TGGACTCGCT GATCCTTGGG AGGGTCTCCT CAGATTGATT GACTGCCCAC  660
   CTCGGGGGTC TTTCATTTGG AGGTTCCACC GAGATTTGGA GACCCCAGCC CAGGGACCAC  720
   CGACCCCCCC GCCGGGAGGT AAGCTGGCCA GCGGTCGTTT CGTGTCTGTC TCTGTCTTTG  780
   TGCGTGTTTG TGCCGGCATC TAATGTTTGC GCCTGCGTCT GTACTAGTTG GCTAACTAGA  840
   TCTGTATCTG GCGGTCCCGC GGAAGAACTG ACGAGTTCGT ATTCCCGGCC GCAGCCCCTA  900
   GGAGACGTCC CAGCGGCCTC GGGGGCCCGT TTTGTGGCCC GTTCTGTGTC GTTAACCACC  960
   CGAGTCGGAC TTTTTGGAGC TCCGCCACTG TCCGAGGGGT ACGTGGCTTT GTTGGGGGAC 1020
   GAGAGACAGA GACACTTCCC GCCCCCGTCT GAATTTTTGC TTTCGGTTTT ACGCCGAAAC 1080
   CGCGCCGCGC GTCTTGTCTG CTGCAGCATC GTTCTGTGTT GTCTCTGTCT GACTGTGTTT 1140
   CTGTATTTGT CTGAAAATTA GGGCCAGACT GTTACCACTC CCTTAAGTTT GACCTTAGAT 1200
   CACTGGAAAG ATGTCGAGCG GATCGCTCAC AACCAGTCGG TAGATGTCAA GAAGAGACGA 1260
   TGGGTTACCT TCTGCTCTGC AGAATGGCCA ACCTTTAACG TCGGATGGCC GCGAGACGGC 1320
   ACCTTTAACC GAGACCTCAT CACCCAGGTT AAGATCAAGG TCTTTTCACC TGGCCCGCAT 1380
   GGACACCCAG ACCAGGTCCC CTACATCGTG ACCTGGGAAG CCTTGGCTTT TGACCCCCCT 1440
   CCCTGGGTCA AGCCCTTTGT ACACCCTAAG CCTCCGCCTC CTCTTCCTCC ATCCGCCCCG 1500
   TCTCTCCCCC TTGAACCTCC TCTTTCGACC CCGCCTCGAT CCTCCCTTTA TCCAGCCCTC 1560
   ACTCCTTCTC TAGGCGGCTC CACCGCGGTG GCGGCCGCTC TAGAACTAGT GGGATCCCGC 1620
   CGCGGCGGGT ACCGAGCTCA TTCGAGTAGC GGCTCTTCCA AGCTCAAAGA AGCAGAGGCC 1680
   GCTGTTCGTT TCCTTTAGGT CTTTCCACTA AAGTCGGAGT ATCTTCTTCC AAGATTTCAC 1740
   GTCTTGGTGG CCGTTCCAAG GAGCGCGAGG TCGGGATGGA TCTTGAAGGG GACCGCAATG 1800
   GAGGAGCAAA GAAGAAGAAC TTTTTTAAAC TGAACAATAA AAGTGAAAAA GATAAGAAGG 1860
   AAAAGAAACC AACTGTCAGT GTATTTTCAA TGTTTCGCTA TTCAAATTGG CTTGACAAGT 1920
   TGTATATGGT GGTGGGAACT TTGGCTGCCA TCATCCATGG GGCTGGACTT CCTCTCATGA 1980
   TGCTGGTGTT TGGAGAAATG ACAGATATCT TTGCAAATGC AGGAAATTTA GAAGATCTGA 2040
   TGTCAAACAT CACTAATAGA AGTGATATCA ATGATACAGG GTTCTTCATG AATCTGGAGG 2100
   AAGACATGAC CAGGTATGCC TATTATTACA GTGGAATTGG TGCTGGGGTG CTGGTTGCTG 2160
   CTTACATTCA GGTTTCATTT TGGTGCCTGG CAGCTGGAAG ACAAATACAC AAAATTAGAA 2220
   AACAGTTTTT TCATGCTATA ATGCGACAGG AGATAGGCTG GTTTGATGTG CACGATGTTG 2280
   GGGAGCTTAA CACCCGACTT ACAGATGATG TCTCTAAGAT TAATGAAGTT ATTGGTGACA 2340
   AAATTGGAAT GTTCTTTCAG TCAATGGCAA CATTTTTCAC TGGGTTTATA GTAGGATTTA 2400
   CACGTGGTTG GAAGCTAACC CTTGTGATTT TGGCCATCAG TCCTGTTCTT GGACTGTCAG 2460
   CTGCTGTCTG GGCAAAGATA CTATCTTCAT TTACTGATAA AGAACTCTTA GCGTATGCAA 2520
   AAGCTGGAGC AGTAGCTGAA GAGGTCTTGG CAGCAATTAG AACTGTGATT GCATTTGGAG 2580
   GACAAAAGAA AGAACTTGAA AGGTACAACA AAAATTTAGA AGAAGCTAAA AGAATTGGGA 2640
   TAAAGAAAGC TATTACAGCC AATATTTCTA TAGGTGCTGC TTTCCTGCTG ATCTATGCAT 2700
   CTTATGCTCT GGCCTTCTGG TATGGGACCA CCTTGGTCCT CTCAGGGGAA TATTCTATTG 2760
   GACAAGTACT CACTGTATTC TTTTCTGTAT TAATTGGGGC TTTTAGTGTT GGACAGGCAT 2820
   CTCCAAGCAT TGAAGCATTT GCAAATGCAA GAGGAGCAGC TTATGAAATC TTCAAGATAA 2880
   TTGATAATAA GCCAAGTATT GACAGCTATT CGAAGAGTGG GCACAAACCA GATAATATTA 2940
   AGGGAAATTT GGAATTCAGA AATGTTCACT TCAGTTACCC ATCTCGAAAA GAAGTTAAGA 3000
   TCTTGAAGGG CCTGAACCTG AAGGTGCAGA GTGGGCAGAC GGTGGCCCTG GTTGGAAACA 3060
   GTGGCTGTGG GAAGAGCACA ACAGTCCAGC TGATGCAGAG GCTCTATGAC CCCACAGAGG 3120
   GGATGGTCAG TGTTGATGGA CAGGATATTA GGACCATAAA TGTAAGGTTT CTACGGGAAA 3180
   TCATTGGTGT GGTGAGTCAG GAACCTGTAT TGTTTGCCAC CACGATAGCT GAAAACATTC 3240
   GCTATGGCCG TGAAAATGTC ACCATGGATG AGATTGAGAA AGCTGTCAAG GAAGCCAATG 3300
   CCTATGACTT TATCATGAAA CTGCCTCATA AATTTGACAC CCTGGTTGGA GAGAGAGGGG 3360
   CCCAGTTGAG TGGTGGGCAG AAGCAGAGGA TCGCCATTGC ACGTGCCCTG GTTCGCAACC 3420
   CCAAGATCCT CCTGCTGGAT GAGGCCACGT CAGCCTTGGA CACAGAAAGC GAAGCAGTGG 3480
   TTCAGGTGGC TCTGGATAAG GCCAGAAAAG GTCGGACCAC CATTGTGATA GCTCATCGTT 3540
   TGTCTACAGT TCGTAATGCT GACGTCATCG CTGGTTTCGA TGATGGAGTC ATTGTGGAGA 3600
   AAGGAAATCA TGATGAACTC ATGAAAGAGA AAGGCATTTA CTTCAAACTT GTCACAATGC 3660
   AGACAGCAGG AAATGAAGTT GAATTAGAAA ATGCAGCTGA TGAATCCAAA AGTGAAATTG 3720
   ATGCCTTGGA AATGTCTTCA AATGATTCAA GATCCAGTCT AATAAGAAAA AGATCAACTC 3780
   GTAGGAGTGT CCGTGGATCA CAAGCCCAAG ACAGAAAGCT TAGTACCAAA GAGGCTCTGG 3840
   ATGAAAGTAT ACCTCCAGTT TCCTTTTGGA GGATTATGAA GCTAAATTTA ACTGAATGGC 3900
   CTTATTTTGT TGTTGGTGTA TTTTGTGCCA TTATAAATGG AGGCCTGCAA CCAGCATTTG 3960
   CAATAATATT TTCAAAGATT ATAGGGGTTT TTACAAGAAT TGATGATCCT GAAACAAAAC 4020
   GACAGAATAG TAACTTGTTT TCACTATTGT TTCTAGCCCT TGGAATTATT TCTTTTATTA 4080
   CATTTTTCCT TCAGGGTTTC ACATTTGGCA AAGCTGGAGA GATCCTCACC AAGCGGCTCC 4140
   GATACATGGT TTTCCGATCC ATGCTCAGAC AGGATGTGAG TTGGTTTGAT GACCCTAAAA 4200
   ACACCACTGG AGCATTGACT ACCAGGCTCG CCAATGATGC TGCTCAAGTT AAAGGGGCTA 4260
   TAGGTTCCAG GCTTGCTGTA ATTACCCAGA ATATAGCAAA TCTTGGGACA GGAATAATTA 4320
   TATCCTTCAT CTATGGTTGG CAACTAACAC TGTTACTCTT AGCAATTGTA CCCATCATTG 4380
   CAATAGCAGG AGTTGTTGAA ATGAAAATGT TGTCTGGACA AGCACTGAAA GATAAGAAAG 4440
   AACTAGAAGG TGCTGGGAAG ATCGCTACTG AAGCAATAGA AAACTTCCGA ACCGTTGTTT 4500
   CTTTGACTCA GGAGCAGAAG TTTGAACATA TGTATGCTCA GAGTTTGCAG GTACCATACA 4560
   GAAACTCTTT GAGGAAAGCA CACATCTTTG GAATTACATT TTCCTTCACC CAGGCAATGA 4620
   TGTATTTTTC CTATGCTGGA TGTTTCCGGT TTGGAGCCTA CTTGGTGGCA CATAAACTCA 4680
   TGAGCTTTGA GGATGTTCTG TTAGTATTTT CAGCTGTTGT CTTTGGTGCC ATGGCCGTGG 4740
   GGCAAGTCAG TTCATTTGCT CCTGACTATG CCAAAGCCAA AATATCAGCA GCCCACATCA 4800
   TCATGATCAT TGAAAAAACC CCTTTGATTG ACAGCTACAG CACGGAAGGC CTAATGCCGA 4860
   ACACATTGGA AGGAAATGTC ACATTTGGTG AAGTTGTATT CAACTATCCC ACCCGACCGG 4920
   ACATCCCAGT GCTTCAGGGA CTGAGCCTGG AGGTGAAGAA GGGCCAGACG CTGGCTCTGG 4980
   TGGGCAGCAG TGGCTGTGGG AAGAGCACAG TGGTCCAGCT CCTGGAGCGG TTCTACGACC 5040
   CCTTGGCAGG GAAAGTGCTG CTTGATGGCA AAGAAATAAA GCGACTGAAT GTTCAGTGGC 5100
   TCCGAGCACA CCTGGGCATC GTGTCCCAGG AGCCCATCCT GTTTGACTGC AGCATTGCTG 5160
   AGAACATTGC CTATGGAGAC AACAGCCGGG TGGTGTCACA GGAAGAGATC GTGAGGGCAG 5220
   CAAAGGAGGC CAACATACAT GCCTTCATCG AGTCACTGCC TAATAAATAT AGCACTAAAG 5280
   TAGGAGACAA AGGAACTCAG CTCTCTGGTG GCCAGAAACA ACGCATTGCC ATAGCTCGTG 5340
   CCCTTGTTAG ACAGCCTCAT ATTTTGCTTT TGGATGAAGC CACGTCAGCT CTGGATACAG 5400
   AAAGTGAAAA GGTTGTCCAA GAAGCCCTGG ACAAAGCCAG AGAAGGCCGC ACCTGCATTG 5460
   TGATTGCTCA CCGCCTGTCC ACCATCCAGA ATGCAGACTT AATAGTGGTG TTTCAGAATG 5520
   GCAGAGTCAA GGAGCATGGC ACGCATCAGC AGCTGCTGGC ACAGAAAGGC ATCTATTTTT 5580
   CAATGGTCAG TGTCCAGGCT GGAACAAAGC GCCAGTGAAC TCTGACTGTA TGAGATGTTA 5640
   AATACTTTTT AATGGGGATC CCCCGGGCTG CAGGAATTCG ATATCAAGCT TATCGATACC 5700
   GTCGACCTCG AGGGGGGGCC CGGTACAGAT TAGTCCAATT TGTTAAAGAC AGGATATCAG 5760
   GTGGTCCAGG CTCTAGTTTT GACTCAACAA TATCACCAGC TGAAGCCTAT AGAGTACGAG 5820
   CCATAGATAG AATAAAAGAT TTTATTTAGT CTCCAGAAAA AGGGGGGAAT GAAAGACCCC 5880
   ACCTGTAGGT TTGGCAAGGC TAGCTTAAGT AAGCCATTTT GCAAGGCATG GAAAAATACA 5940
   TAACTGAGAA TAGAGAAGTT CAGATCAAGG TTAGGAACAG AGAGACAGGA GAATATGGGC 6000
   CAAACAGGAT ATCTGTGGTA AGCAGTTCCT GCCCCGGCTC AGGGCCAAGA ACAGTTGGAA 6060
   CAGCAGAATA TGGGCCAAAC AGGATATCTG TGGTAAGCAG TTCCTGCCCC GGCTCAGGGC 6120
   CAAGAACAGA TGGTCCCCAG ATGCGGTCCC GCCCTCAGCA GTTTCTAGAG AACCATCAGA 6180
   TGTTTCCAGG GTGCCCCAAG GACCTGAAAT GACCCTGTGC CTTATTTGAA CTAACCAATC 6240
   AGTTCGCTTC TCGCTTCTGT TCGCGCGCTT CTGCTCCCCG AGCTCAATAA AAGAGCCCAC 6300
   AACCCCTCAC TCGGCGCGCC AGTCCTCCGA TAGACTGCGT CGCCCGGGTA CCCGTATTCC 6360
   CAATAAAGCC TCTTGCTGTT TGCATCCGAA TCGTGGACTC GCTGATCCTT GGGAGGGTCT 6420
   CCTCAGATTG ATTGACTGCC CACCTCGGGG GTCTTTCAGT AGGATCTCGA CCGATGCCCT 6480
   TGAGAGCCTT CAACCCAGTC AGCTCCTTCC GGTGGGCGCG GGGCATGACT ATCGTCGCCG 6540
   CACTTATGAC TGTCTTCTTT ATCATGCAAC TCGTAGGACA GGTGCCGGCA GCGCTCTGGG 6600
   TCATTTTCGG CGAGGACCGC TTTCGCTGGA GCGCGACGAT GATCGGCCTG TCGCTTGCGG 6660
   TATTCGGAAT CTTGCACGCC CTCGCTCAAG CCTTCGTCAC TGGTCCCGCC ACCAAACGTT 6720
   TCGGCGAGAA GCAGGCCATT ATCGCCGGCA TGGCGGCCGA CGCGCTGGGC TACGTCTTGC 6780
   TGGCGTTCGC GACGCGAGGC TGGATGGCCT TCCCCATTAT GATTCTTCTC GCTTCCGGCG 6840
   GCATCGGGAT GCCCGCGTTG CAGGCCATGC TGTCCAGGCA GGTAGATGAC GACCATCAGG 6900
   GACAGCTTCA AGGATCGCTC GCGGCTCTTA CCAGCCTAAC TTCGATCACT GGACCGCTGA 6960
   TCGTCACGGC GATTTATGCC GCCTCGGCGA GCACATGGAA CGGGTTGGCA TGGATTGTAG 7020
   GCGCCTGATG CGGTATTTTC TCCTTACGCA TCTGTGCGGT ATTTCACACC GCATATGGTG 7080
   CACTCTCAGT ACAATCTGCT CTGATGCCGC ATAGTTAAGC CAGCCCCGAC ACCCGCCAAC 7140
   ACCCGCTGAC GCGCCCTGAC GGGCTTGTCT GCTCCCGGCA TCCGCTTACA GACAAGCTGT 7200
   GACCGTCTCC GGGAGCTGCA TGTGTCAGAG GTTTTCACCG TCATCACCGA AACGCGCGAG 7260
   ACGAAAGGGC CTCGTGATAC GCCTATTTTT ATAGGTTAAT GTCATGATAA TAATGGTTTC 7320
   TTAGACGTCA GGTGGCACTT TTCGGGGAAA TGTGCGCGGA ACCCCTATTT GTTTATTTTT 7380
   CTAAATACAT TCAAATATGT ATCCGCTCAT GAGACAATAA CCCTGATAAA TGCTTCAATA 7440
   ATATTGAAAA AGGAAGAGTA TGAGTATTCA ACATTTCCGT GTCGCCCTTA TTCCCTTTTT 7500
   TGCGGCATTT TGCCTTCCTG TTTTTGCTCA CCCAGAAACG CTGGTGAAAG TAAAAGATGC 7560
   TGAAGATCAG TTGGGTGCAC GAGTGGGTTA CATCGAACTG GATCTCAACA GCGGTAAGAT 7620
   CCTTGAGAGT TTTCGCCCCG AAGAACGTTT TCCAATGATG AGCACTTTTA AAGTTCTGCT 7680
   ATGTGGCGCG GTATTATCCC GTATTGACGC CGGGCAAGAG CAACTCGGTC GCCGCATACA 7740
   CTATTCTCAG AATGACTTGG TTGAGTACTC ACCAGTCACA GAAAAGCATC TTACGGATGG 7800
   CATGACAGTA AGAGAATTAT GCAGTGCTGC CATAACCATG AGTGATAACA CTGCGGCCAA 7860
   CTTACTTCTG ACAACGATCG GAGGACCGAA GGAGCTAACC GCTTTTTTGC ACAACATGGG 7920
   GGATCATGTA ACTCGCCTTG ATCGTTGGGA ACCGGAGCTG AATGAAGCCA TACCAAACGA 7980
   CGAGCGTGAC ACCACGATGC CTGTAGCAAT GGCAACAACG TTGCGCAAAC TATTAACTGG 8040
   CGAACTACTT ACTCTAGCTT CCCGGCAACA ATTAATAGAC TGGATGGAGG CGGATAAAGT 8100
   TGCAGGACCA CTTCTGCGCT CGGCCCTTCC GGCTGGCTGG TTTATTGCTG ATAAATCTGG 8160
   AGCCGGTGAG CGTGGGTCTC GCGGTATCAT TGCAGCACTG GGGCCAGATG GTAAGCCCTC 8220
   CCGTATCGTA GTTATCTACA CGACGGGGAG TCAGGCAACT ATGGATGAAC GAAATAGACA 8280
   GATCGCTGAG ATAGGTGCCT CACTGATTAA GCATTGGTAA CTGTCAGACC AAGTTTACTC 8340
   ATATATACTT TAGATTGATT TAAAACTTCA TTTTTAATTT AAAAGGATCT AGGTGAAGAT 8400
   CCTTTTTGAT AATCTCATGA CCAAAATCCC TTAACGTGAG TTTTCGTTCC ACTGAGCGTC 8460
   AGACCCCGTA GAAAAGATCA AAGGATCTTC TTGAGATCCT TTTTTTCTGC GCGTAATCTG 8520
   CTGCTTGCAA ACAAAAAAAC CACCGCTACC AGCGGTGGTT TGTTTGCCGG ATCAAGAGCT 8580
   ACCAACTCTT TTTCCGAAGG TAACTGGCTT CAGCAGAGCG CAGATACCAA ATACTGTCCT 8640
   TCTAGTGTAG CCGTAGTTAG GCCACCACTT CAAGAACTCT GTAGCACCGC CTACATACCT 8700
   CGCTCTGCTA ATCCTGTTAC CAGTGGCTGC TGCCAGTGGC GATAAGTCGT GTCTTACCGG 8760
   GTTGGACTCA AGACGATAGT TACCGGATAA GGCGCAGCGG TCGGGCTGAA CGGGGGGTTC 8820
   GTGCACACAG CCCAGCTTGG AGCGAACGAC CTACACCGAA CTGAGATACC TACAGCGTGA 8880
   GCATTGAGAA AGCGCCACGC TTCCCGAAGG GAGAAAGGCG GACAGGTATC CGGTAAGCGG 8940
   CAGGGTCGGA ACAGGAGAGC GCACGAGGGA GCTTCCAGGG GGAAACGCCT GGTATCTTTA 9000
   TAGTCCTGTC GGGTTTCGCC ACCTCTGACT TGAGCGTCGA TTTTTGTGAT GCTCGTCAGG 9060
   GGGGCGGAGC CTATGGAAAA ACGCCAGCAA CGCGGCCTTT TTACGGTTCC TGGCCTTTTG 9120
   CTGGCCTTTT GCTCACATGT TCTTTCCTGC GTTATCCCCT GATTCTGTGG ATAACCGTAT 9180
   TACCGCCTTT GAGTGAGCTG ATACCGCTCG CCGCAGCCGA ACGACCGAGC GCAGCGAGTC 9240
   AGTGAGCGAG GAAGCGGAAG AGCGCCCAAT ACGCAAACCG CCTCTCCCCG CGCGTTGGCC 9300
   GATTCATTAA TGCAGNNG                                              9318

## Beschreibung

Die Erfindung betrifft retrovirale Vektorhybride und deren Verwendung zum Gentransfer, insbesondere für den Gentransfer in hämatopoietischen Stammzellen (ES).

Replikationsdefekte retrovirale Vektoren sind gegenwärtig Standard in Gentransfer-und Gentherapieapplikationen an Zellen des menschlichen blutbildenden Systems (A.D. Miller (1992) *(1)*, R.C. Mulligan (1993) *(2)*, R. Vile und S.J. Russell (1994) *(3)*). Ihre wesentlichen Vorteile sind:
― die Infektion führt zu einer stabilen Integration des Vektors und der durch ihn übertragenen DNA-Sequenzen in das Wirtsgenom mitotisch aktiver Zellen
― die Anzahl der Integrationen in das Wirtsgenom ist über die Infektionsbedingungen steuerbar
― die sicherheitsrelevanten Aspekte des retroviralen Gentransfers sind gut erforscht; Komplikationen traten bei zahlreichen Anwendungen am Menschen bisher nicht auf.

Der meistgenannte Vorteil von retroviralen Vektoren, die hohe Gentransfereffizienz, trifft auf die Anwendung am blutbildenden System nur bedingt zu. Mit konventionellen, auf dem Moloney murine sarcoma Virus (MoMuSV) basierenden Vektoren lassen sich bevorzugt nur späte, ausreifende blutbildende Vorläuferzellen infizieren; 30-95% der mit diesen Vektoren transduzierten Vorläuferzellen zeigen infolge primärer Silencing-Mechanismen keine oder unzureichende Expression der übertragenen Gene (4); nach längerer Verweildauer in vivo wird zudem in einem hohen Prozentsatz der initial exprimierenden Zellen die retroviral gesteuerte Genexpression bis zur Funktionsunfähigkeit durch sekundäres Silencing abgeschwächt (Palmer et al. (1991) *(5)*, Brenner et al. (1993) *(6)*).

Es hat sich gezeigt, daß auch in embryonalen Zellen, wie embryonalen Carcinomazellen (myeloischen (nicht lymphatischen) Abkömmlingen von hämatopoietischen Stammzellen), die Viren zwar integriert werden, aber die Expression des integrierten Provirus geblockt wird.

Aus Stocking et al. (1993) (21) sind Mutationen und Derivate von MoMuSV bekannt, mit denen die retrovirale Genexpression in derartigen Zellen verbessert werden kann. Solche Hybride werden durch Punktmutationen in den LTRs, insbesondere in der U3-Region erhalten. Beispielsweise kann durch eine Punktmutation bei -345 von MoMuLV die Bindung des ECF-1 Repressors verringert werden. Ebenso vorteilhaft ist eine Punktmutation, welche eine Bindungsstelle für SP-1 schafft (z.B. Punktmutation bei -166 (S. McKnight und R. Tijan (1986) *(16)*). Weiter vorteilhaft ist es, auch außerhalb der LTR-Bereiche Mutationen anzubringen. Es hat sich gezeigt, daß eine 18 bp Region, die sich direkt downstream an das 5'-LTR anschließt, ein negatives Regulationselement (NRE, Silencerbindungselement) darstellt. Durch Punktmutationen in diesem Element ist es möglich, die retrovirale Transkription zu verbessern (murines embryonales Stammzellvirus, MESV; M. Grez et al (1990) *(22)*).

Die Verwendung eines modifizierten Harvey murine sarcoma virus (Ha-MuSV), dessen U3-Regionen in beiden LTRs durch komplementäre Regionen aus dem Friend murine leukemia virus (F-MuLV) ersetzt wurden, wurde von Velu et al. (1991) (42) als effizienter retroviraler Vektor beschrieben. Dieses Konstrukt weist gegenüber dem Ha-MuSV ohne Modifikation eine bereits verbesserte Expressionsrate in hematopoietischen Stammzellen auf.

Aufgabe der Erfindung ist es, retrovirale Vektorhybride zur Verfügung zu stellen, mit denen die retrovirale Genexpression insbesondere in hämatopoietischen Stammzellen und ihren myeloischen (nicht-lymphatischen) Abkömmlingen weiter verbessert werden kann.

Gegenstand der Erfindung ist ein retrovirales Vektorhybrid, das dadurch gekennzeichnet ist, daß es
a) in der Leaderregion als US-Region und tRNA primer binding site die US-Region und tRNA primer binding site von MESV und
b) als U3- und R-Regionen in 3'-LTR, die U3- und R-Regionen aus einem Friend murine leukemia virus (F-MuLV) oder aus Myeloproliferative Sarkomavirus (MPSV)
enthält.

Ebenso bevorzugt enthalten die erfindungsgemäßen Vektorhybride als 3'-LTR das LTR aus einem Friend murine leukemia virus (F-MULV) und damit, außer den U3- und R-Regionen aus MESV, auch die U5-Region aus F-MuLV.

Die Vektorhybride können sowohl replikationsdefekt als auch replikationskompetent sein.

Unter einem replikationsdefekten Vektor ist ein Vektor zu verstehen, der keine retroviralen Gen-Funktionen (gag, env, pol) enthält und dadurch nicht selbständig Viruspartikel bilden kann. Üblicherweise enthält der Vektor jedoch eine packaging-Funktion (psi). Zur Bildung von infektiösen retroviralen Partikeln wird eine packaging-Zellinie, welche die Gen-Funktionen gag, env und pol stabil (als Episom oder ins Genom integriert) enthält, mit dem replikationsdefekten erfindungsgemäßen DNA-Vektor transfiziert. Es werden RNA-Transkripte gebildet, die aufgrund der gag- und env-Funktionen in Viruspartikel verpackt werden. Diese Viruspartikel sind replikationsdefizient, weil das in ihnen enthaltene RNA-Genom keine retroviralen Gen-Funktionen trägt.

Replikationsfähige, retrovirale Vektorhybride enthalten zusätzlich die Genregionen gag, pol und env. Diese Genregionen können aus beliebigen Retroviren stammen. Lediglich die env-Region muß, entsprechend der zu infizierenden Zelle, ausgewählt werden. Derartige Verfahren sind dem Fachmann jedoch bekannt. Auf diese Weise sind replikationsfähige ecotrope, xenotrope, amphotrope oder polytrope Retroviren herstellbar.

Näheres hierzu sowie zur Herstellung von Vektoren, Viruspartikeln und Helferzellen ist beschrieben in M. Kriegler, Gene Transfer and Expression, A Laboratory Manual, W.H. Freeman & Co., New York (1990), 47-55 und 161-164) (*31*), worauf hiermit ausdrücklich Bezug genommen wird.

Unter Silencerproteinen im Sinne der Erfindung sind zelluläre (z.B. aus der mutierten Zelle stammende) nukleäre Proteine, beispielsweise aus hämatopoietischen Stammzellen, zu verstehen, welche vorzugsweise an die unmittelbar dem 5'-LTR folgenden, ersten 450 bp, vorzugsweise die ersten 18 bp, von MoMuLV binden und durch Interaktion mit cis-regulatorischen Sequenzen des Provirus die retrovirale Transkription inhibitieren.

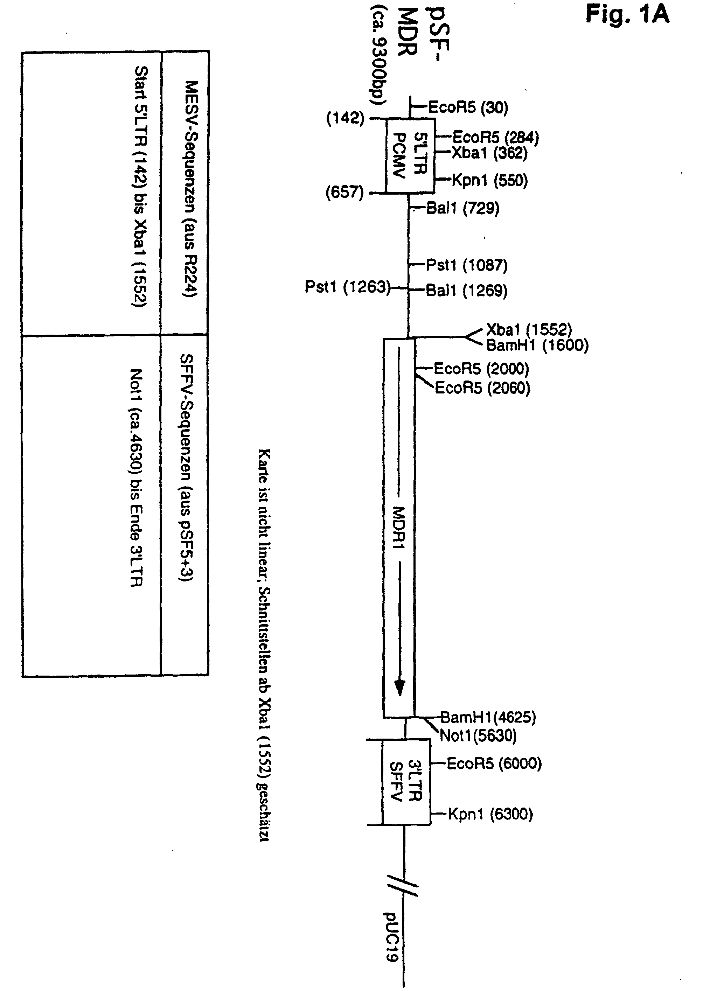

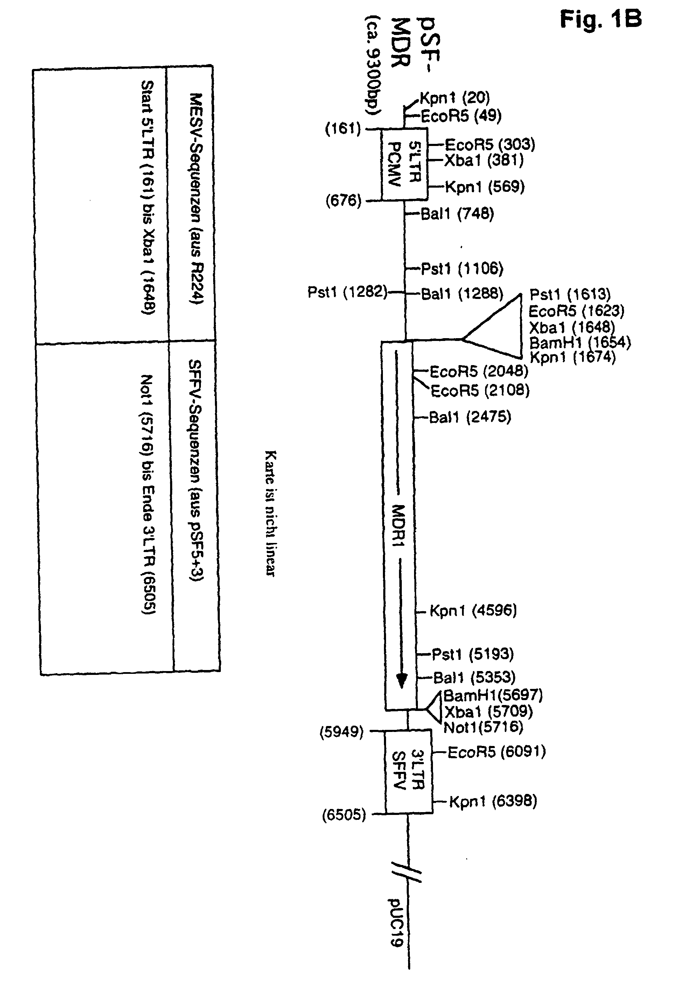

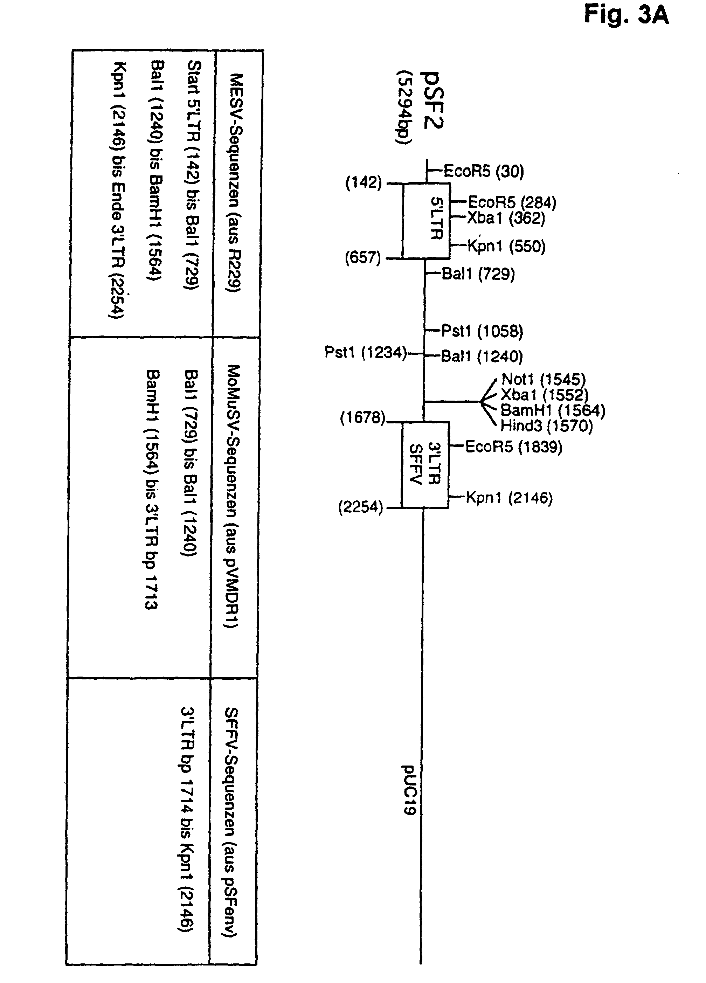

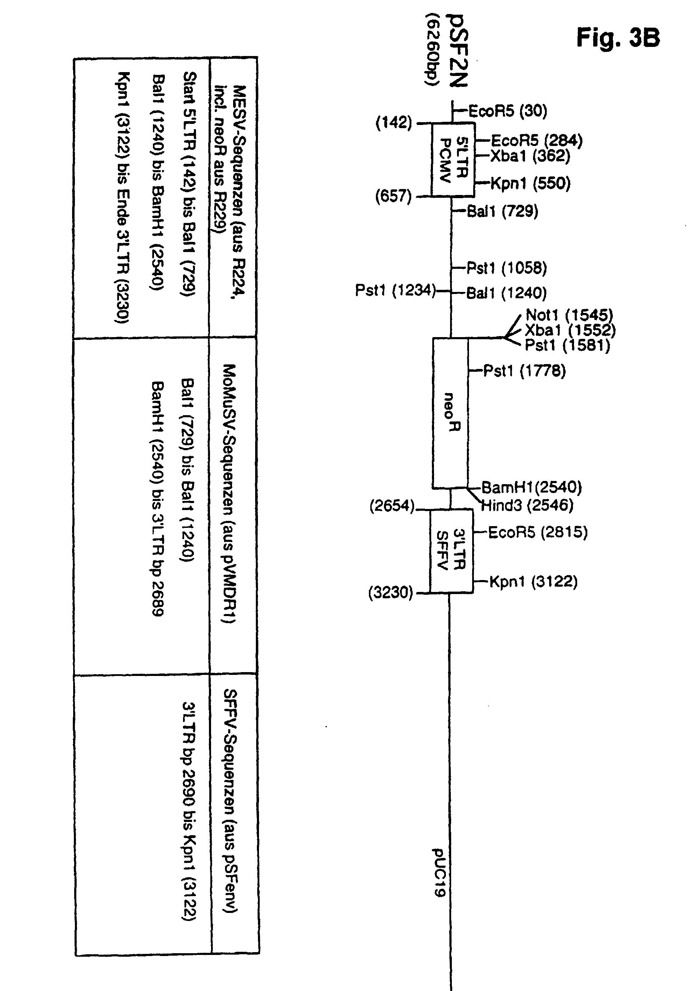

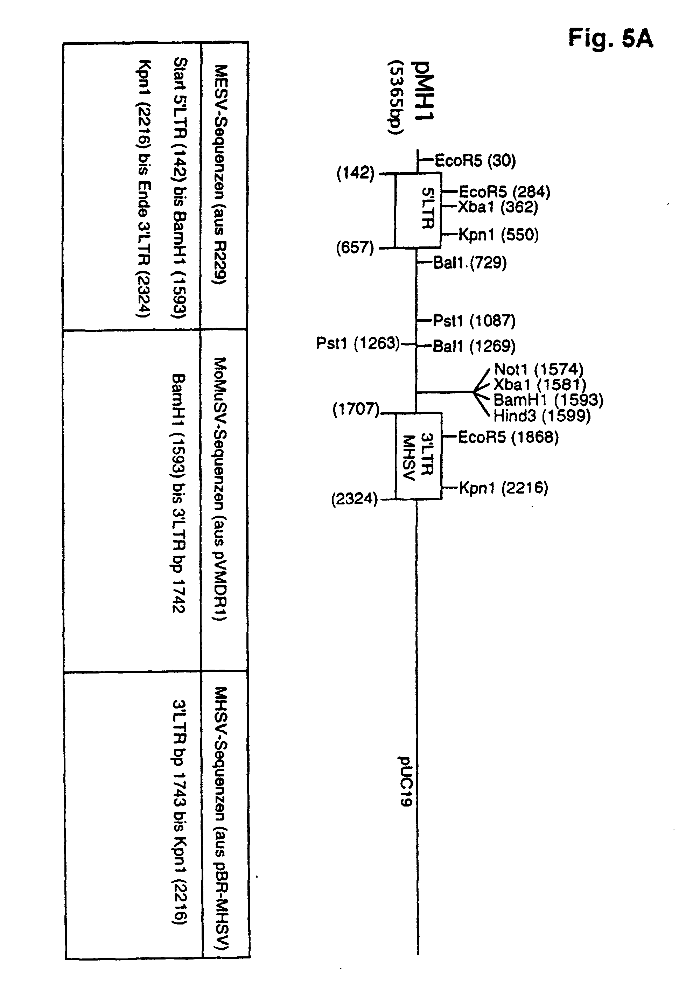

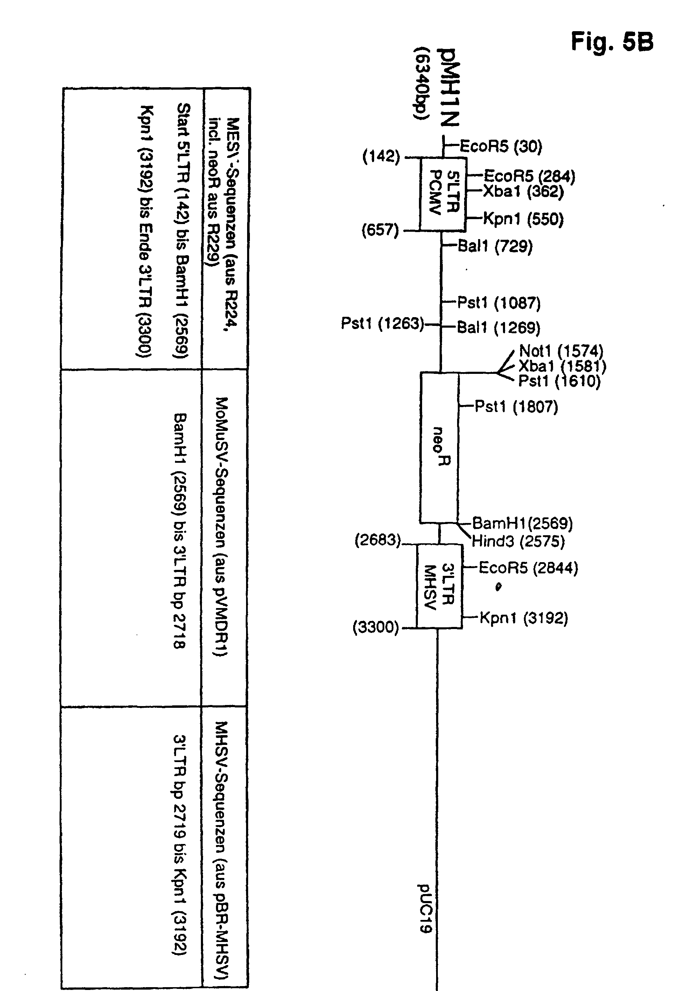

Unter Leaderregion von MESV ist die Summe von Sequenzen aus dem kurzen direkten terminalen Repeat (R), der US-Region und der unmittelbar an das 5'-LTR von MESV anschließenden Sequenz zu verstehen, welche sowohl die tRNA primer binding site als auch die packaging-Region (ψ) enthält. Die Leadersequenz kann maximal aus der vollständigen Sequenz bestehen, die im Virus von R bis zum Translationsstart enthalten ist. Vorzugsweise wird jedoch eine verkürzte Leaderregion verwendet, die neben R und U5 vorzugsweise aus in etwa den ersten 450 Basen downstream vom Ende der 5'-LTR-Region besteht. Ebenso ist es bevorzugt, daß ausschließlich oder im wesentlichen die U5-Region und die tRNA primer binding site von MESV und die anderen Teile der Leadersequenz aus MoMuLV oder MoMuSV stammen. Ein geeigneter MESV-Bereich ist in den Abbildungen (Fig. 1 - 5) von KpnI (ca. 550) bis BalI (ca. 729) gezeigt.

Die Leaderregion von MESV entspricht der Leaderregion von MoMuLV oder MoMuSV (Sequenz von U3, U5 und R von MoMuLV und PCMV, siehe *(22)* und *(23)*), in der Punktmutationen eingefügt sind, mit denen die Bindung von Silencerproteinen vermindert oder verhindert wird. Die Leaderregion von MESV enthält gegenüber MoMuLV und PCMV in der 18 bp Region, welche sich direkt an das 5'-LTR anschließt, 5 Punktmutationen (C. Stocking et al. (1993) *(21)*). Dadurch wird die inhibitorische Bindungsfähigkeit von Kernproteinen aus den infizierten Zellen drastisch vermindert und die Expression eines im Vektorhybrid enthaltenen Gens entsprechend verbessert (R. Petersen et al., Mol. Cell. Biol. 11 (1991) 1213-1221 *(8)*). Anstelle dieser Punktmutationen sind auch andere (mindestens eine) Punktmutationen in der Leaderregion (vorzugsweise innerhalb der ersten 450 Basen downstream vom Ende der 5'-LTR-Region, und besonders bevorzugt in der 18 bp Region) geeignet, die inhibitorische Bindung von Silencerproteinen z.B. an die Leaderregion zu vermindern oder zu verhindern. Damit ist im Sinne der Erfindung unter einer Leaderregion von MESV außer der obengenannten und in SEQ ID NO:1 beschriebenen Sequenz auch eine Sequenz zu verstehen, die im wesentlichen diesem Abschnitt von SEQ ID NO:1 entspricht, eine tRNA-Bindungsstelle und eine psi-Funktion enthält und durch Punktmutationen so modifiziert ist, daß die inhibitorische Bindung von Silencerproteinen aus den Zellen, die zur Transfektion vorgesehen sind, vermindert oder verhindert wird.

Zur Überprüfung, ob eine Punktmutation geeignet ist, die Bindung von Silencerproteinen an die Leaderregion zu verhindern, wird üblicherweise ein Plasmid hergestellt, welches ein LTR, die zu überprüfende Leaderregion und ein Indikatorgen, vorzugsweise das CAT-Gen, enthält. Die zu überprüfenden Zellen werden mit diesem Plasmid transfiziert und die CAT-Aktivität nach vorzugsweise 48 Stunden bestimmt (transiente Transfektion). Ist die CAT-Aktivität deutlich vorhanden, so ist die Punktmutation geeignet, die Bindung des Silencerproteins in diesen Zellen zu verhindern (P. Artelt et al. (1991) *(32)*).

Mit transienten Transfektionsexperimenten zeigt sich, daß in der Leadersequenz von MoMuSV cis-regulatorische Sequenzen lokalisiert sind, die einen inhibitorischen Einfluß auf die Genexpression insbesondere in frühen, multipotenten myeloischen Zellen haben (primäres Silencing). Austausch dieses Bereiches gegen Leader-sequenzen des murinen embryonalen Stammzellvirus [MESV *(22)*] hebt den inhibitorischen Einfluß in myeloischen Stammzellen vollständig auf. Die Leader-Sequenz von MESV geht zurück auf das endogene Mausretrovirus dl-587rev *(23)* und wurde in MESV eingeführt, um das am MoMuSV-Leader beobachtete Silencing in embryonalen Stammzellen aufzuheben *(22).* Entscheidend für die Aufhebung des Silencings sind Mutationen der retroviralen primer binding site (PBS), die unmittelbar 3' des 5'LTRs gelegen ist *(22)*.

Retroviren der F-MuLV-Gruppe sind dem Fachmann bekannt und beispielsweise in D. Linnemeyer et al. (1981) *(7)*, J. Friel et al. (1990) *(9),* S.P. Clark und T.W. Mak (1982) *(10),* L. Wolff et al. (1985) *(11)*, R.K. Bestwick et al. (1984) *(12)*, W. Koch et al. (1984) *(13)* und A. Adachi et al. (1984) *(14)* beschrieben. Eine Übersicht findet sich in W. Ostertag et al. (1987) *(15).* Vorzugsweise werden die 3'-LTRs vom malignen Histiosarkomvirus (MHSV) *(9)*, SFFVp Lilly-Steeves *(10)*, SFFVa *(11)*, Rauscher SFFV *(12),* F-MuLV c157 *(13)* und Friend-mink cell focus forming Virus (F-MCFV FrNx *(14)*, F-MCFV pFM548 *(13)*) verwendet.

Erfindungsgemäß sind als 3'-LTR Sequenzen der genomischen Vektor-RNA geeignet, welche in der U3-Region eine hochgradige Sequenzhomologie zu den 3'-LTR Bereichen der obengenannten Friend-Virus-Familie zeigen. Die LTR-Sequenz von SFFVp ist in SEQ ID NO: 1 beschrieben (Nucleotide 1707 - 2283).

Die erfindungsgemäßen Vorteile zeigen sich bei transienten Transfektionen von Reportergenkonstrukten in einer Vielzahl von repräsentativen Zellinien von Mensch und Maus. Es hat sich überraschenderweise gezeigt, daß U3-Sequenzen von Mausretroviren der Friend murine leukemia virus (F-MuLV) Familie in Kombination mit Leadersequenzen (vorzugsweise tRNA binding site) von MESV eine insbesondere effiziente Genexpression in myeloischen Stamm-und Vorläuferzellen ermöglichen. Die Steigerung der Genexpression mit erfindungsgemäßen Vektorhybriden gegenüber MoMuSV beträgt mehr als eine Zehnerpotenz. Dies trifft sowohl für relativ späte Vorläuferzellen der Granulozyten, Makrophagen und Erythrozyten als auch für frühe, multipotente Vorläufer und auch Stammzellen des myeloischen Sytems zu. Insbesondere bei den unreifen, multipotenten myeloischen Zellen, bei denen MoMuSV-LTR ein ausgeprägtes primäres Silencing zeigt, erlauben die erfindungsgemäßen Vektorhybride eine effiziente Genexpression. Auch im lymphatischen System sowie in Fibroblasten ist die Aktivität hoch.

Besonders bevorzugt ist ein frühes Isolat des Polyzythämie-induzierenden spleen focus forming virus (SFFVp *(7)*), dessen LTR-Sequenz in SEQ ID NO:1 (bp 2654-3230) beschrieben ist, sowie das maligne Histiosarkomvirus (MHSV (9), LTR SEQ ID NO:4, bp 1707-2324). In der U3-Region dieser Viren besteht eine hochgradige Sequenzhomologie zu anderen Vertretern der Friendvirus-Familie wie SFFVp Lilly-Steeves *(10)*, SFFVa *(11),* Rauscher SFFV *(12)*, F-MuLV c157 *(13)* und Friend-mink cell focus forming Virus [F-MCFV FrNₓ *(14)*, F-MCFV pFM548 *(13)*]. Diese und ähnliche Friend-verwandte Retroviren weisen aufgrund der Sequenzhomologie der U3-Regionen ein ähnliches Expressionsmuster wie SFFVp und MHSV auf.

Besonders vorteilhaft für die im Vergleich zu MoMuSV ausgeprägte Steigerung der Genexpression in myeloischen Stamm- und Vorläuferzellen durch die erfindungsgemäßen Vektoren sind Mutationen in der Enhancerregion von U3 [340 bis 140 Nukleotide 5' des Transkriptionsstarts gelegen *(10)*]. Von Bedeutung ist die partielle Duplikation des sogenannten direct-repeat Elements bei SFFVp (ähnlich F-MCFV FrNx) und MHSV. Möglicherweise sind hier Bindungsstellen für Transkriptionsfaktoren lokalisiert, die in myeloischen Zellen für effiziente Genexpression sorgen.

Unmittelbar 5' des direct repeats tragen SFFVp und MHSV sowie Friend MCFV eine Bindungsstelle für den Transkriptionsfaktor Spl *(16).* Spl ist zu multiplen Interaktionen mit umgebenden Transkriptionsfaktoren fähig, die in der Regel zu einer erheblichen Steigerung der Genexpression führen. Bei den MoMuSV-Derivaten Myeloproliferatives Sarkomvirus (MPSV *(17), (18))* und PCC4-Zell passagiertes MPSV (PCMV *(19))* ist das Auftreten einer Sp1-Bindungsstelle in der U3-Region außerdem notwendig für die Aufhebung des Silencings des MoMuSV-LTRs in embryonalen Stammzellen *(20)* sowie wahrscheinlich auch in Blutstammzellen (21). Eine analoge Funktion könnte auch der Sp1-Bindungsstelle von SFFVp, MHSV sowie Friend-MCF-Viren zukommen.

In einer weiteren Ausführungsform der Erfindung können die retroviralen Vektorhybride als U3- und R-Regionen in 3'-LTR die U3- und R-Regionen aus MPSV enthalten. MPSV unterscheidet sich von MoMuSV bzw. MoMuLV durch Punktmutationen im LTR *(21)*. Dabei sind A(-381) deletiert und die Austausche C→T, -345; T→A, -326; T→A, -249 und A→C, -166 (Bezifferung gemäß *(21)*) durchgeführt. In diesem Fall enthält das retrovirale Vektorhybrid in der Leaderregion als U5-Region und tRNA primer binding site die U5-Region und tRNA primer binding site von MESV. Es hat sich gezeigt, daß derartige MPSV/MESV-Hybridvektoren (im weiteren auch mit MPEV bezeichnet) deutliche Vorteile gegenüber Vektoren auf Basis von MoMuLV besitzen und von großer Bedeutung für eine Vielzahl von Anwendungen in der somatischen Gentherapie sind.

In 5'-LTR können beliebige U3- und R-Regionen verwendet werden, da nach der Integration des Virus das U3 aus 3'-LTR in der Zielzelle nach Ablauf des retroviralen Lebenszyklus auch an die 5'-LTR-Position kopiert wird und die Genexpression treibt. U3- und R-Regionen stammen beispielsweise aus MoMuLV- oder MoMuSV-Derivaten, wie beispielsweise MPSV, PCMV und MESV. Ebenso geeignet als 5'-LTR sind 5'-LTRs von F-MuLV.

Die erfindungsgemäßen Vektorhybride zeigen nach retroviraler Transduktion von myeloischen Stamm- und Vorläuferzellen eine hohe gewebsspezifische Expression. Diese Vektoren haben daher das Potential, die Gentransfereffizienz in myeloische Stamm-und Vorläuferzellen gegenüber MoMuSV-Vektoren deutlich zu erhöhen. Die erfindungsgemäßen Vektoren haben außerdem das Potential, in wesentlich geringerem Ausmaß Silencingvorgängen in myeloischen Zellen unterworfen zu sein als MoMuSV-Vektoren. Die hohe und eventuell auch persistierende Genexpression dieser Vektoren in myeloischen Zellen kann die Grundlage schaffen für die erfolgreiche Applikation zahlreicher Gentransferprotokolle am blutbildenden System des Menschen. Die in den Beispielen beschriebenen Konstrukte sind so aufgebaut, daß die vom Vektor übertragenen cDNAs problemlos durch andere Gene ersetzt werden können.

Die Gewebspezifität der Expression ist geeignet, das bei konventionellen MoMuSV-Vektoren beobachtete *primäre* Silencing im myeloischen System aufzuheben. Dies führt zu einer deutlichen Steigerung der funktionellen Gentransferraten in myeloische Zellen. Da eine hohe funktionale Gentransferrate bei den meisten Gentransfer-/Gentherapie-applikationen am myeloischen System erwünscht ist, liegt hier ein generell nutzbarer Vorteil der erfindungsgemäßen Konstrukte.

Die Friendvirus-verwandten U3-Regionen wurden im myeloischen Sytem der Maus auch auf Persistenz der Genexpression in vivo selektioniert. *Sekundäres* Silencing tritt mit diesen Vektoren in wesentlich geringerem Ausmaß auf als bei MoMuSV-Vektoren. Die Exklusion von inhibitorischen Leadersequenzen durch Austausch gegen MESV-Sequenzen wird diesbezüglich zusätzlich von Vorteil sein. Dies ist von Bedeutung für alle Gentransfer-/Gentherapieapplikationen, bei denen eine langanhaltende (womöglich lebenslange) Expression der retroviral übertragenen Sequenzen im myeloischen System erwünscht ist (Genmarkierungsstudien, Korrektur metabolischer Defekte).

Hierfür können die erfindungsgemäßen Vektorhybride mindestens ein für das Virus heterologes, in eukaryontischen Zellen exprimierbares Gen enthalten. Derartige Gene sind beispielsweise das multiple drug resistance-Gen (MDR-Gen), ein Antibiotikaresistenzgen wie das neo^{R}-Gen, das LNGFR-Gen, das Cerebrosidasegen oder das Herpes simplex TK-Gen. In einer bevorzugten Ausführungsform können auch mehrere, vorzugsweise zwei oder drei heterologe Gene im Vektorhybrid enthalten sein. Um eine Expression dieser getrennten Gene zu ermöglichen, wird zweckmäßig vor das zweite bzw. dritte Gen ein Promotor, eine splicing site (vorzugsweise von SF7Vp) oder eine internal ribosomal entry site (IRES, vorzugsweise von Polio-Viren (I.R. Ghattas et al. (1991) *(26)*)) eingefügt.

Die Erzeugung hoher Virustiter in fibroblastoiden Verpackungszellinien ist eine weitere wichtige Anforderung an retrovirale Vektoren, die für den Gentransfer in myeloische Zellen eingesetzt werden sollen. Die Aktivität der Friend-verwandten U3-Regionen in fibroblastischen Retrovirus-Verpackungszellinien reicht bei den unten aufgeführten Beispielkonstrukten in jedem Fall aus, die erforderlichen Titer von 10⁵ bis 10⁶ vektorübertragenden retroviralen Parkikeln/ml Zellkulturüberstand zu erzeugen.

Gegenstand der Erfindung ist demnach auch ein Verfahren zur Herstellung einer retroviral transduzierten, eukaryontischen Zelle, wobei humane embryonale Stammzellen ausgeschlossen sind, welche ein aktives exogenes Gen enthält, welches dadurch gekennzeichnet ist, daß die eukaryontische Zelle transduziert wird mit einem replikationsdefekten, retroviralen Vektorvirus, der in seinem Genom
a) in der Leaderregion als US-Region und tRNA primer binding site die U5-Region und die tRNA primer binding site von MESV,
b) als U3- und R-Regionen in 3'-LTR, die U3- und R-Regionen aus einem Friend murine leukemia virus (F-MuLV) oder Myeloproliferative Sarkomavirus (MPSV) und
c) das genannte exogene Gen enthält.

Vorzugsweise wird als 3'-LTR das LTR aus F-MuLV verwendet, welches dann die genannten U3- und R-Regionen enthält.

Ein weiterer Gegenstand der Erfindung sind retroviral transduzierte, eukaryontische Zellen, wobei humane embryonale Stammzellen ausgeschlossen sind, dadurch erhältlich, daß die eukaryontische Zelle transduziert wird mit einem replikationsdefekten, retroviralen Vektorvirus, der in seinem Genom
a) in der Leaderregion als US-Region und tRNA primer binding site die U5-Region und die tRNA primer binding site von MESV,
b) als U3- und R-Regionen in 3'-LTR, die U3- und R-Regionen aus einem Friend murine leukemia virus (F-MuLV) oder Myeloproliferative Sarkomavirus (MPSV) enthält.
Gegebenenfalls enthält dieser Vektor ein oder mehrere (bis zu drei) exogene Gene, die in der eukaryontischen Zelle exprimierbar sind. Vorzugsweise werden als eukaryontische Zellen Säugerzellen, vorzugsweise hämatopoietische Zellen, insbesondere hämatopoietische Stammzellen verwendet.

Vorzugsweise wird als 3'-LTR das LTR aus F-MuLV verwendet, welches dann die genannten U3- und R-Regionen enthält.

Unter einem aktiven exogenen Gen ist ein Gen zu verstehen, welches von außen in die Zelle eingebracht wird und in dieser Zelle nach Integration ins Genom exprimiert wird (aktiv ist). Das exogene Gen kann ein im Zellgenom nicht enthaltenes Gen (z.B. ein Antibiotikaresistenzgen wie neo^{R}, LNGFR-Rezeptor (D. Johnson et al. (1986) (33)), das Cerebrosidase-Gen oder das Herpes simplex TK-Gen) sein oder ein im Genom enthaltenes, dort aber nicht oder nur ungenügend exprimiertes Gen sein, wie das multiple drug resistance (MDR) gene.

Ein weiterer Gegenstand der Erfindung ist ein replikationsdefektes, infektiöses Viruspartikel, welches als Genom eine retrovirale RNA enthält, wobei das Genom eine Leaderregion aus MESV enthält, welche eine packaging-Funktion und eine t-RNA-Bindungsstelle enthält, ein in einer eukaryontischen Zelle exprimierbares und für den Virus heterologes Gen und am 3'-Ende U3 und R aus einem Friend murine leukemia virus (F-MuLV), jedoch keine aktiven gag-, envund pol-Sequenzen enthält.

Ein weiterer Gegenstand der Erfindung ist ein Verfahren zur Herstellung eines replikationsdefekten, infektiösen Viruspartikels durch Transfektion einer eukaryontischen Helferzelle, weiche die Helferfunktionen gag, env und pol besitzt, mit einem Vektorhybrid, welches als Leaderregion die Leaderregion von MESV und als 3'-LTR das LTR aus einem Friend murine leukemia virus (F-MuLV) oder Myeloproliferative Sarkomavirus (MPSV) enthält, Produktion von der DNA des Vektorhybrids entsprechender RNA als Virusgenom in der Zelle (beispielsweise durch zelluläre Polymerasen), Verpackung der genannten RNA in in der Zelle entstandene, replikationsdefiziente, leere Virushüllen und Isolation der infektiösen Viruspartikel, welche das genannte Virusgenom enthalten.

Ein weiterer Gegenstand der Erfindung ist die Verwendung eines erfindungsgemäßen replikationsdefekten, retroviralen Vektorhybrids, welches in der Leaderregion als U5-Region und tRNA primer binding site die U5-Region und die tRNA primer binding site von MESV und als U3- und R-Regionen in 3'LTR, die U3- und R-Regionen aus einem Friend murine leukemia virus (F-MuLV) oder aus Myeloproliferative Sarkomavirus (MPSV) enthält, zur Herstellung eines Arzneimittels zur ex vivo oder in vivo Gentherapie.

### Anwendungsgebiete

### I. Alle somatischen Gentransfer-/Gentherapieverfahren, bei denen myeloische Stamm-und Vorläuferzellen Zielpopulation für retrovirale Vektoren sind.

Die individuellen Protokolle werden jeweils unterschiedliche cDNAs in die Vektoren integrieren. Beispiele sind
- Genmarkierungsstudien *(1)*: Selektionierbare Markergene wie neoR oder verkürzter nerve growth factor receptor werden übertragen um das Schicksal der markierten Zellpopulation im Organismus unter den Voraussetzungen der jeweils untersuchten Erkrankung/Therapie zu verfolgen. Die neoR-Vektoren pSF1N und pMH1N, die neoR unter Kontrolle des SFFVp U3 bzw. MHSV U3 tragen (MESV-Leader) zeigen im Vergleich zu neoR-übertragenden Moloney-Vektoren eine deutliche Steigerung der funktionalen Gentransferrate im Modellsystem der Maus-Stammzellinie FDCPmix. Sie sind für den neoR-Transfer in humane myeloische Stammzellen geeignet.
- Schutz des Knochenmarks gegen die Nebenwirkungen einer Hochdosis-Chemotherapie *(28)*: Chemotherapieresistenz-vermittelnde Gene wie MDR1 oder Alkyltransferasen werden übertragen. Hohe Transferrate und Expression über die optimierten Friendvirus-verwandten Vektoren verhindern myelosuppressive Nebenwirkungen und evtl. auch Zweittumorinduktion der Chemotherapie.
- Korrektur metabolischer Erkrankungen: Bei monogenen Erbleiden werden über retroviralen Gentransfer in myeloische Stammzellen intakte Kopien der defekten Gene eingebracht. Anwendungen sind z.B. denkbar bei Erbleiden, deren Gendefekt Auswirkungen im myeloischen Sytem hat (Speicherkrankheiten wie Hurler-Syndrom; M. Gaucher *(29)*):

### II. Klonierung replikationskompetenter Friend-verwandeter Retroviren

Die erfindungsgemäßen Viren sind aufgrund ihres in Richtung auf frühe myeloische Stamm-und Vorläuferzellen erweiterten Wirtsspektrums besonders gut für die Insertionsmutagenese in diesen Zellen geeignet. Über Insertionsmutagenese lassen sich Gene klonieren, die eine wichtige Funktion bei der physiologischen Wachstumsregulation des myeloischen Sytems haben.

Das Prinzip, Retroviren als Insertionsmutagen zu verwenden, wurde schon mehrfach beschrieben (Review: Kung et al. (1991) *(34)*). Retroviren integrieren DNA-Sequenzunspezifisch ins Genom und können dabei Gene aktivieren oder inaktivieren. Replizierbare Retroviren infizieren Gewebe mit einer sehr hohen Effizienz und können daher die Wahrscheinlichkeit steigern, daß durch Integration Mutationen erzeugt werden. Solche Mutationen können wiederum zu einer Entartung der mutierten Zellen führen, d.h. Tumoren entstehen.

Bei in vivo Experimenten mit Mäusen (gewöhnlich an neugeborenen Mäusen, da sie noch kein effizient arbeitendes Immunsystem haben und daher gegen Retroviren sehr empfindlich sind) zur Identifizierung regulatorischer Gene werden jene mit Retroviren infiziert, und nach einer Latenzperiode von ca. 3 Monaten können spezifische Tumoren (abhängig vom Virustyp) entdeckt und untersucht werden. Analysen der klonalen, retroviralen Integrationsstellen enthüllen Gene, deren Aktivierung bzw. Inaktivierung zu Zellen veränderten bzw. entarteten Phänotypes führen kann. Damit ist es möglich, eine große Zahl verschiedenster Gen-Typen zu entdecken, wie Gene für Liganden, Rezeptoren, Signal-Transduktoren und Transkriptionsfaktoren. Signifikaterweise existiert eine hohe Korrelation zwischen dem gefundenen Tumortypus, der durch bestimmte Viren erzeugt wird, und dem aktivierten/inaktivierten Gen (z.B. bei der Erythroleukämie, die durch Friend-MuLV induziert wird, zeigen ca. 80% der Tumoren Integrationsereignisse im Fli-1 Locus und transkriptionelle Aktivierung des Transkriptionsfaktor PU-1-Gens: Interagierende Oncogene konnten identifiziert werden, indem transgene Mäuse mit ecotroper Expression eines Oncogens (z.B. *myc*) infiziert und die Tumorgenese beobachtet wurde. Nach deutlich verkürzter Latenzzeit entstanden Tumoren durch Sekundärmutationen ("Second Hit", z.B. im *pim-1-*Gen)).

Dennoch konnte mit bisherigen Retroviruskonstrukten lediglich ein Ausschnitt an möglichen Genen identifiziert werden. Das Spektrum an Genen, das mit diesem Ansatz, durch Aktivierung/Inaktivierung und die damit verbundene, phänotypische Veränderung der Zelle, auffindbar ist, hängt stark von der Spezifität der verwendeten Retroviren für bestimmte Zelltypen ab. So zeigen bisher verwendete Retroviren nur sehr geringe Infektiösität in hämatopoietischen und embryonalen Stammzellen (vgl. Review: Stocking et al. (1993) *(21)*). Dies ist auf mehrere Ursachen zurückführbar; primär auf Transkriptionskontrollelemente in den retroviralen LTRs (Stocking et al. (1985) *(18)*), sekundär aber auch auf, für die Virusreplikation notwendige, Proteininteraktionen zwischen zellulären und viralen Proteinen.

Replikationskompetente Retroviren erzeugen durch Infektion neue Retrovirusproduzentenzellen, was zu einer vollständigen Infektion der gesamten Zellpopulation und mit einer weit größeren Wahrscheinlichkeit zur Ausbildung von Mutationen führt. Ähnliche Effizienz läßt sich nur durch eine aufwendigere und zeitraubendere Cokultivierung der Retrovirusverpackungslinie mit der Zielzellinie erreichen.

Friend-verwandte replikationskompetente Viren können außerdem zur Erzeugung von forcedpassage-Mutanten eingesetzt werden, die eine zusätzliche Verbesserung der Infizierbarkeit von Blutstammzellen aufweisen. Mutationen in retroviralen Strukturproteinen dieser Viren können Verwendung finden bei der Erzeugung optimierter Verpackungszellinien für den Gentransfer in myeloische Zellen. Mutationen in cis-regulatorischen Sequenzen dieser Viren können eingesetzt werden zur weiteren Optimierung retroviraler Vektoren für den Blutstammzellgentransfer.

Ein weiterer Gegenstand der Erfindung sind replikationsfähige, ecotrope, xenotrope, amphotrope oder polytrope Retroviren, mit den regulatorischen Elementen (Leaderregion) von MESV, kombiniert mit U3- und R-Regionen in 3'-LTR, die U3- und R-Regionen aus einem Friend murine leukemia virus (F-MuLV), die dadurch in der Lage sind, murine (in vivo und in vitro) oder humane (in vitro) Vorläuferzellen effizienter zu infizieren und Mutationen auszulösen. Vorzugsweise enthalten die Retroviren als 3'-LTR das LTR aus F-MuLV.

Die Kombination beider Komponenten führt zu retroviralen Insertionsmutagenesekonstrukten einer neuen Qualität, mit deren Hilfe neue, mit herkömmlichen, replikationsfähigen Retroviren nicht auffindbare Gene, identifiziert werden können. Sie sind mit einem sehr hohen Titer erzeugbar und ergeben eine nahezu 100 %ige Infektionsrate, da jede befallene Zelle zur Produzentenzelle für neue Viren wird. In ihnen kann das Neomycin-Resistenzgen, das in retroviralen Vektoren zur Selektion Verwendung findet, deletiert werden. Dies führt zu einer höheren Transkription der LTR-Promotoren, da neo^{R} als Silencer funktioniert (Artelt et al. (1991) *(32)*), und demzufolge zu einer verbesserten Insertionsrate.

Durch die erfindungsgemäßen Retroviren können murine als auch humane hämatopoietische Stammzellen in vitro mit hoher Effizienz infiziert und durch Insertion ins Genom Mutanten (Aktivierung bzw Inaktivierung von Genen) erzeugt werden. Es ist daher auch für in vivo-Ansätze zu erwarten, daß die erfindungsgemäßen Viren frühe hämatopoietische Zellen mit höherer Effizienz infizieren und Gene durch Integration ins Genom aktivieren bzw. inaktivieren.

Eine Genaktivierung kann auf mehrere Weisen passieren. So kann eine Integration stromaufwärts eines Genes jenes unter der transkriptionellen Kontrolle des 3'-LTR-Promotors aktivieren. Es kann auch die Bindungsstellen von negativen Kontrollelementen inaktiviert werden, was zu einer Genaktivierung führen kann. Es existieren aber auch mehrere Beispiele (Kung et al. (1991) *(34)*), die belegen, daß der 5'-LTR-Promotor direkt über Durchlesen oder aber über seine Enhancer-Wirkung auch viele Basen stromaufwärts, aber auch innerhalb eines Introns, nach erfolgter Insertion, ein Gen aktivieren kann.

Eine Integration innerhalb eines Genes oder in der zugehörigen Promotor/Enhancer-Region kann aber auch zu einer Inaktivierung des entsprechenden Genes führen.

Sind für die Regulation der Zelle wichtige Gene betroffen, können die Zellen entarten, d.h. es können Tumoren entstehen. Mit Hilfe der erfindungsgemäßen Viren können daher andere Gene, z.B. Gene, welche bei der Zellregulation von hämatopoietischen Stammzellen involviert sind, gefunden werden, als mit herkömmlichen Viren, welche in jenen Zelltypen nicht oder nur sehr schlecht replizieren können.

Die erfindungsgemäßen replikationskompetenten Viren können in vitro beispielsweise folgendermaßen angewendet werden:

Faktorenabhängige, promyelocytische Zellinien wurden durch retrovirale Insertionsmutagenese faktorunabhängig gemacht, d.h. das Wachstumsfaktorgen wurde durch retrovirale Integration aktiviert. Die Retrovirusinfektion erfolgte durch Cokultivierung der Zielzellinie mit MPSV-Vektor produzierenden Verpackungslinien. Dieser in vitro-Ansatz läßt sich mit Hilfe der erfindungsgemäßen Konstrukte effizienter gestalten. Des weiteren sind mit den erfindungsgemäßen Viren murine oder humane, hämatopoietische Stammzellen obigen Untersuchungen zugänglich.

Eine weitere Anwendungsmöglichkeit besteht in Experimenten, die das Ziel haben, bessere Vektorvirusverpackungslinien z.B. für die Gentherapie herzustellen, die Viren mit einer höheren Infektionseffizienz in Vorläuferzellen produzieren können. In jenen Zellen existieren mehrere Blocks, die mit der retroviralen Infektion/Replikation interferieren. Die Verwendung der erfindungsgemäßen Konstrukte hat gegenüber replikationsfähigen Wildtyp MoMuLV oder AM4070 den Vorteil, daß die transkriptionellen Blocks bereits beseitigt sind. Verbesserungen in der Infektionseffinzienz lassen sich daher auf entsprechende Mutationen in den viralen Proteinen zurückführen.

Eine weitere Anwendungsmöglichkeit der erfindungsgemäßen Konstrukte liegt in der Erzeugung von "Knock Out"-Mäusen durch Insertionsinaktivierung eines oder mehrerer Gene in murinen embroyonalen Stammzellen.

Die folgenden Beispiele, Zeichnungen und Sequenzprotokolle erläutern die Erfindung weiter.

Die Sequenzprotokolle zeigen:
- **SEQ ID NO:1**: Sequenz von pSF1
- **SEQ ID NO:2**: Sequenz von pSF2
- **SEQ ID NO:3**: Sequenz von pSF3
- **SEQ ID NO:4**: Sequenz von pMH1
- **SEQ ID NO:5**: Sequenz von pSF-MDR
- **SEQ ID NO:6**: Sequenz von pMP-MDR
(dargestellt ist jeweils nur der provirale Teil der Sequenzen)
- **Fig. 1A**: zeigt die Plasmidkarte von pSF-MDR.
(orientierende Angabe der Restriktionsstellen)
- **Fig. 1B**: zeigt die Plasmidkarte von pSF-MDR
(genaue Angabe der Restriktionsstellen)
- **Fig. 2A**: zeigt die Restriktionskarte von pSF1.
- **Fig. 2B**: zeigt die Restriktionskarte von pSF1N. Dieser Vektor entspricht pSF1, in den in die multiple cloning site (MCS) das neo^{R} Gen insertiert wurde.
- **Fig. 3A**: zeigt die Restriktionskarte von pSF2.
- **Fig. 3B**: zeigt die Restriktionskarte von pSF2N, welche pSF2 nach Integration des neo^{R} Gens entspricht.
- **Fig. 4A**: zeigt die Restriktionskarte von pSF3.
- **Fig. 4B**: zeigt die Restriktionskarte von pSF3N, welche pSF3 nach Integration des neo^{R} Gens entspricht.
- **Fig. 5A**: zeigt die Restriktionskarte von pMH1.
- **Fig. 5B**: zeigt die Restriktionskarte von pMH1N, welches pMH1 nach Integration des neo^{R} Gens entspricht.
- **Fig. 6**: zeigt das Entstehungsschema des Vektors pSF-MDR1.
- **Fig. 7**: zeigt das Entstehungsschema der Vektoren pSF1N, pSF2N, pSF3N und pMH1N.

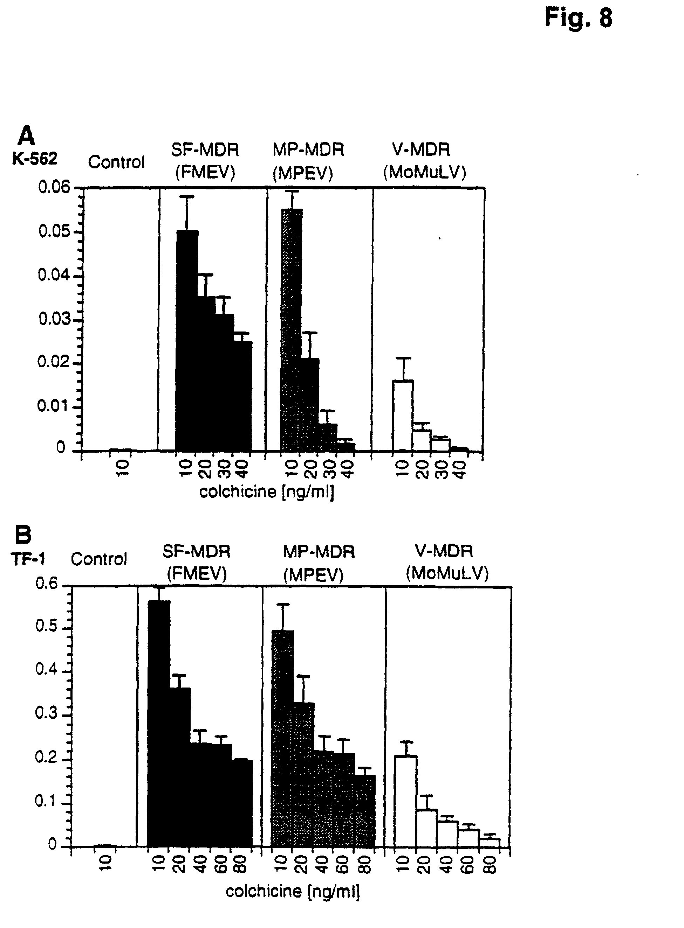

- **Fig. 8**: zeigt die Protektion von myeloiden Zellen durch FMEV und MPEV-mdrI-Vektoren im Vergleich zu Standard MoMuL V-Vektoren.
Angegeben ist die mittlere relative Transduktionshäufigkeit (korrigiert um Fibroblastentiter und Klonierungseffizienz). Fibroblastentiter variierten um weniger als Faktor 3 zwischen den verschiedenen Vektoren. A: Zellinie K-562, B: Zellinie TF-1.
- **Fig. 9**: zeigt die Restriktionskarte von pMP-MDR.

### Beispiel 1

### a) Konstruktionsbeschreibung

Tabelle 1 zeigt die Klonierungsstrategien verschiedener Konstrukte.

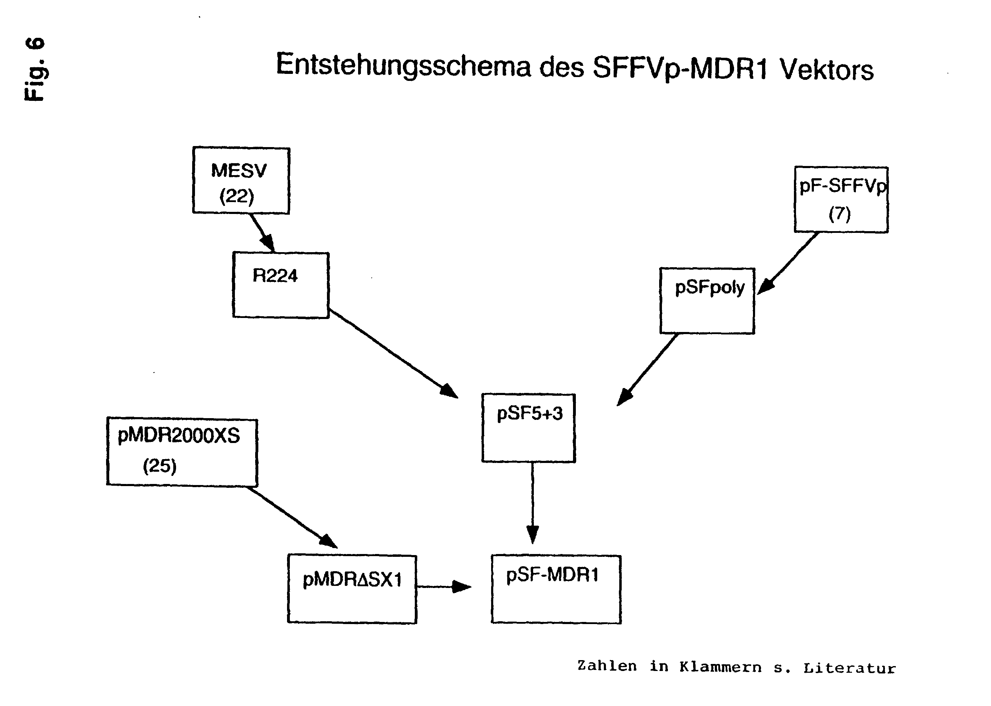

### I. pSF-MDR (Fig. 1, 6, Tabelle 1, SEQ ID NO:5)

Dieser Vektor wurde kloniert auf der Basis des MESV-Vektors R224 (Basis pUC 19), bei dem Teile der env-Region sowie die komplette U3-Region des 3'LTR durch SFFVp-Sequenzen ersetzt wurden. Auf diese Weise wurde der Vektor pSF5 + 3 (vgl. Fig. 6) erhalten. In einem zweiten Schritt erfolgte die Insertion der für MDR1 kodierenden cDNA (aus pMDR2000XS (25)) in die multiple cloning site (Not1, Xba1, BamH1, Hind3).

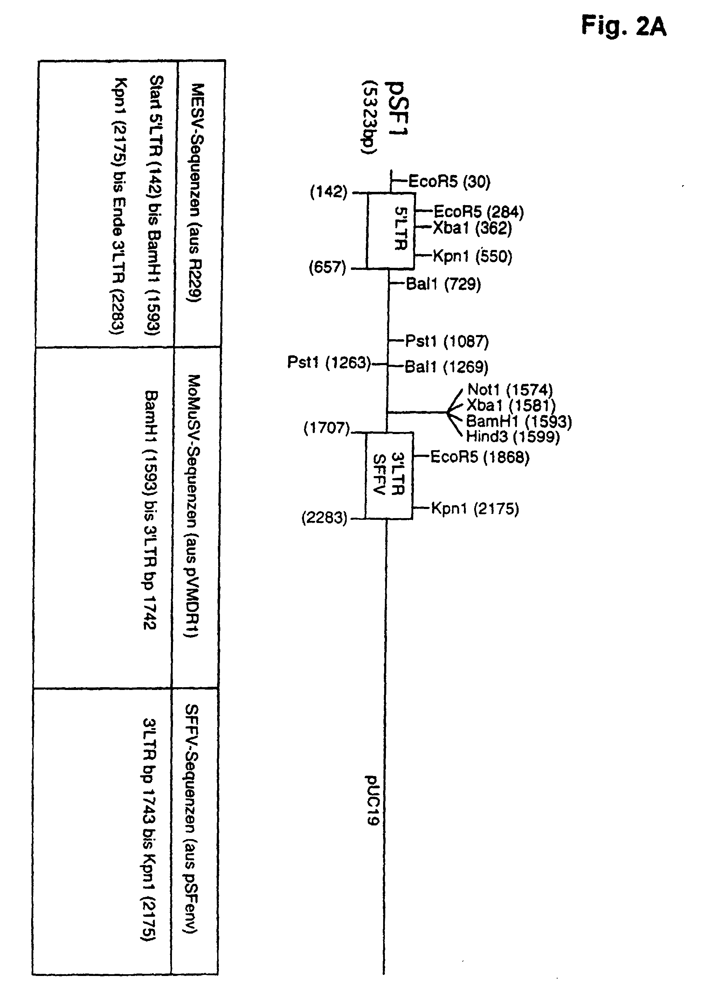

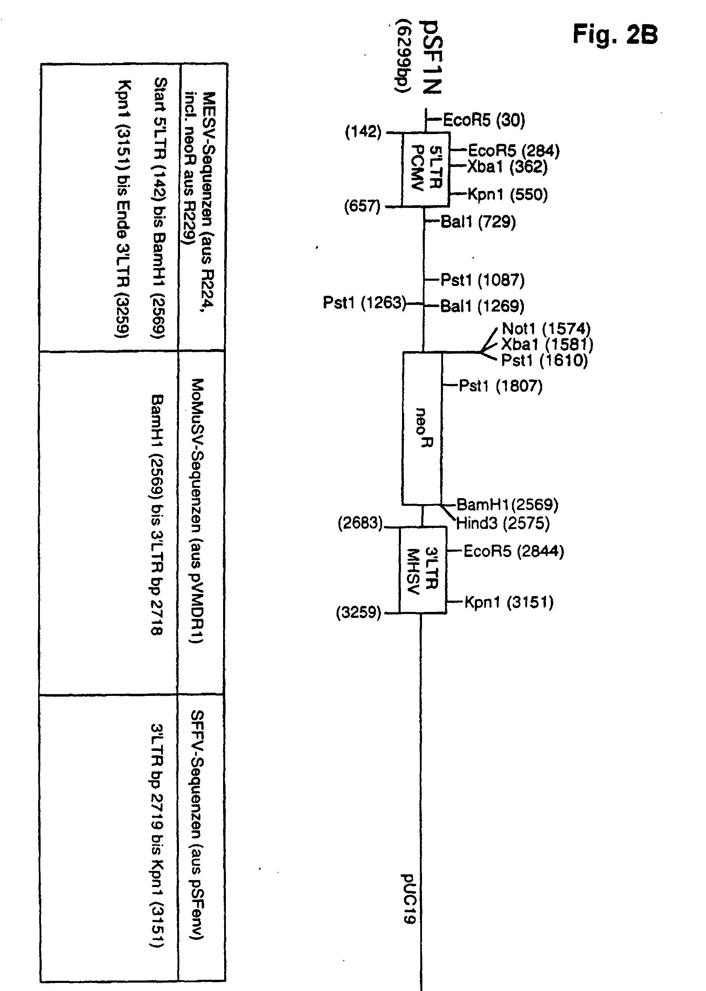

### II. pSF1N, pSF2N, pSF3N, pMH1N (Fig. 2-5, 7, Tabelle 1)

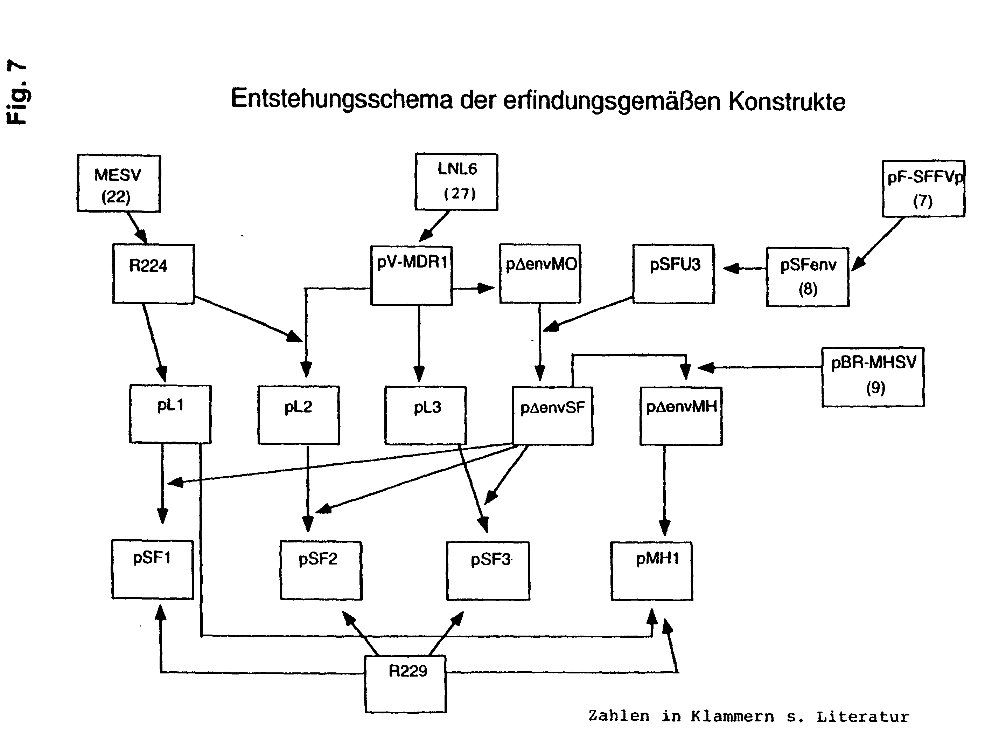

Das Rückgrat dieser Plasmide basiert auf dem MESV-Vektor R224, bei dem eine große Deletion zwischen der Xbal-Schnittstelle des 5'LTR und der Kpnl-Schnittstelle im 3'LTR vorgenommen wurde. Über Dreifragmentligationen wurden die komplementierenden Sequenzen eingeführt; es resultierten die Basisvektoren pSF1, pSF2, pSF3 und pMH1(Entstehungsschema und Klonierungsstrategie, Fig. 7, Tabelle 1, Sequenzen in SEQ ID NO:1-4 beschrieben). Die für die Dreifragmentligation eingesetzten Fragmente werden aus Hilfskonstrukten gewonnen (s. Entstehungsschema, Fig. 6), in denen leicht Modifikationen zur weiteren Optimierung der Vektoren vorgenommen werden können. Denkbar sind Austausch der SFFVp bzw. MHSV U3-Regionen gegen analoge Sequenzen anderer Friend-verwandter Retroviren oder die oben erwähnten Modifikationen des Leaders zur Titeroptimierung. Nach Insertion der neoR cDNA (aus R229) entstanden die Beispielkonstrukte pSF1N, pSF2N, pSF3N, pMH1N. Die Beispielkonstrukte tragen Polylinker mit singulären Restriktionsschnittstellen, die zum Austausch der zu transferierenden Gene verwendet werden können. An diesen Schnittstellen können außerdem regulatorische Sequenzen für eine eventuell notwendige Zweitgenexpression wie der splice acceptor von SFFVp oder die sog. interne Ribosomeneintrittsstelle IRES (I.R. Ghattas et al. (1991) *(26)*) eingeführt werden.

Die in den Beispielkonstrukten als U3 und R klonierten Friend-verwandten Regionen (im 3'LTR) sind nach Ablauf eines retroviralen Lebenszyklus auch an die 5'LTR-Position kopiert und treiben dann die Genexpression in den Zielzellen. Die Beispielkonstrukte enthalten alle für retroviralen Gentransfer und retrovirale Genexpression notwendigen cis-regulatorischen Elemente. Bei diesen Konstrukten sind Eigenschaften von SFFVp (bzw. MHSV), MESV und MoMuSV kombiniert. Als sicherheitsrelevante Modifikationen sind integriert die Punktmutation des Startcodons für das retrovirale gag-Protein zu einem Stopcodon (A.D. Miller und G.J. Rosman (1990) *(27)*) sowie -bei pSF1N, pSF2N, pSF3N, pMHlN- die Deletion überflüssiger env-Sequenzen *(27).*

**Tabelle 1**

| Klonierungsstrategien verschiedener Konstrukte und Hilfskonstrukte. | | | |
|---|---|---|---|
| Konstrukt | Rückgrat | Fragment 1 | Fragment 2 |
| **pSFMDR** | SF5+3 Sal1/not1 | pMDRSX1 Sal1/Not1 | |
| pSF5+3 | R224ΔNB Xho1/Nru1 | pSFpoly Hmd3 blunt end | |
| pMDRΔSX1 | Bluescript KS Sal1/Sma1 | pMDR2000XS Sal1/Ssp1 | |
| **pSF1N** | pSF1 Not1/BamH1 | neoR Not1/BamH1 (R229) | |
| **pSF2N** | pSF2 Not1/BamH1 | neoR Not1/BamH1 (R229) | |
| **pSF3N** | pSF3 Not1/BamH1 | neoR Not1/BamH1 (R229) | |
| **pMH1N** | pMH1 Not1/BamH1 | neoR Not1/BamH1 (R229) | |
| pSF1 | R229ΔXba1/Kpn1 | pL1 Xba1/Not1 | pΔenvSF Not1/Kpn1 |
| pSF2 | R229ΔXba1/Kpn1 | pL2 Xba1/Not1 | pΔenvSF Not1/Kpn1 |
| pSF3 | R229ΔXba1/Kpn1 | pL3 Xba1/Not1 | pΔenvSF Not1/Kpn1 |
| pMH1 | R229ΔXba1/Kpn1 | pL1 Xba1/Not1 | pΔenvMH Not1/Kpn1 |
| pL1 | Bluescript KS Xba1/Not1 | R224 Xba1/Not1 | |
| pL2 | pL1ΔBall | pV-MDR1 Bal1 | |
| pL3 | pL1ΔKpn1-Pst1 | pV-MDR1 kpn1/Pst1 | |
| pΔenvMO | Bluescript KS BamH1/Kpn1 | pV-MDR1 BamH1/Kpa1 | |
| pΔenvSF | pΔenvMO Nhe1/Kpn1 | pSFU3 Xba1/Kpn1 | |
| pΔenvMH | pΔenvSF EcoR5/Kpn1 | pBR-MHSV EcoR5/Kpn1 | |
| pSFU3 | pUC19 Hinc2 | pSFenv SfaN1/Kpn1 | |

### Beispiel 2

### Steigerung der funktionalen Gentransferrate gegenüber konventionellen Moloney-Vektoren

Das multiple drug resistance 1 (MDR1) Gen kodiert für eine membranständige Effluxpumpe (P-Glycoprotein), die Resistenz gegen eine Reihe klinisch wichtiger Zytostatika vermittelt (I. Pastan et al. (1988) *(25)*). Der Grad der Resistenz ist eng an die Expressionshöhe von P-Glycoprotein geknüpft. Gelingt es, myeloische Stammzellen durch retroviralen MDR1-Transfer zytostatikaresistent zu machen, könnte ein wichtiger Beitrag zur Senkung der Nebenwirkungsrate einer Tumor-Chemotherapie geleistet werden.

In dem Experiment wurden K562-Zellen (ATCC CLL243) und TF1-Zellen (humane myeloide Progenitorzellinien) mit retroviralen Vektoren infiziert, die MDR1 unter Kontrolle des SFFV-LTRs exprimieren (Virus SF-MDR hervorgegangen aus Konstrukt pSF-MDR mit MESV-Leader sowie MPSV/MESV-Hybridvektor pMP-MDR).

Die TF1-Zellinie wurde wie bei T. Kitamura et al. (1989) *(30)* beschrieben von einem Patienten mit Erythroleukämie erhalten. Die Zellen werden in RPMI-Medium (supplementiert mit 20% fötalem Kälberserum, 1 mM Na-Pyruvat, 4 mM Glutamin und 20 U/ml rekombinantem GM-CSF) in Kultur gehalten.

In einem identischen Ansatz erfolgte die Infektion von K562-Zellen und TF1-Zellen mit einem Moloney-MDR1 Vektor (Virus V-MDR hervorgegangen aus dem Konstrukt pVMDR), der in einem klinischen Gentransferprotokoll unter Leitung von Prof. A. Deisseroth am MD Anderson Cancer Center, Houston, Texas, eingesetzt wird. Wie aus Tabelle 2 ersichtlich ist, führt SF-MDR bei K562 zu einer ca. 20fachen Steigerung der Gentransferrate gegenüber VMDR, gemessen an der Zahl der unter Zytostatikaapplikation (ca. 10fache LD50 bei *K562)* wachsenden Kolonien.

Die Erhöhung der funktionalen Gentransferrate bei myeloischen Zellen ist Ausdruck der durchschnittlichen Erhöhung der Genexpressionsrate in den transduzierten Zellen. Dies ist von Bedeutung bei Gentransferapplikationen, deren Erfolg auch von der Höhe der Genexpression abhängt (s. Anwendungsbeispiel: Knochenmarksschutz bei Hochdosis-Chemotherapie). Werden bei dem oben beschriebenen Experiment (Infektion von humanen myeloischen Zellen mit SFMDR oder V-MDR) die Zellen unter sehr hohen Zytostatikadosen plattiert (ca. 20fache LD50 bei TF1), sind nur SF-MDR infizierte Zellen in der Lage, Kolonien zu bilden (Tabelle 2).

**Tabelle 2**

| Kolonienzahl unter Colchicinselektion nach Infektion von humanen hämatopoerischen Zellen K562 und TF1 durch MDR1-transduzierende retrovirale Vektoren. Die Vektoren pSF, MDR und pMP-MDR steigern die funktionelle Gentransferrate im Vergleich zum konventionellen MoMuLV-Vektor deutlich. | | | | |
|---|---|---|---|---|
| | | | Kolonienzahl unter Selektion | |
| Virus | U3-Region von ^{3'}LTR | Leader | K562 (20 ng Colchicin) | TF1 (80 ng Colchicin) |
| SF-MDR | SFFVₚ | MESV | 948 +/- 98 | 13 +/- 3 |
| V-MDR | MoMuLV | MoMuSV | 38 +/- 4 | 0 |
| MP-MDR | MPSV | MESV | 410 ± 28 | 9 ± 3 |

Die Zellen wurden mit retroviralem Überstand von amphotropen Verpackungszellinien infiziert. Der Titer auf Fibroblasten war für beide Vektoren zum Zeitpunkt des Experiments ca. 2x10⁴/ml. Das Verhältnis Vektor/Zielzelle war bei der Infektion <1. 24 Stunden nach Infektion wurden 5x10⁴ Zellen in Colchicinhaltigem Weichagarmedium plattiert. Die Auswertung erfolgte am Tag 8 der Agarkloierung. Die Klonierungseffizienz ohne Selektion betrug für beide Zellinien ca. 15%. Die Experimente erfolgten in Duplikaten.

Für einen Vektor, dessen U3-Region aus MoMuLV und dessen Leader aus MESV stammt, werden analoge Ergebnisse wie mit dem Vektor pV-MDR erhalten.

Im MDR1-System haben Konstrukte mit einem MESV-Leader gegenüber konventionellen MoMuSV-Vektoren etwas reduzierte Titer. Dies könnte auf Mutationen im Packagingbereich von MESV zurückzuführen sein. Hybride zwischen den Leadersequenzen von MESV und MoMuSV haben das Potential, Vektoren mit höherem Titer aufgrund verbesserter Verpackungsfunktion in retroviralen Verpackungszellinien unter gleichzeitigem Ausschluß cisinhibitorischer Sequenzen zu erzeugen. Dies wurde bei der Klonierung der Beispielkonstrukte pSF2N und pSF3N berücksichtigt.

### Anmerkung: Provirale Vektoren werden z. B. mit SF-MDR, MP-MDR etc. bezeichnet, um sie von den korrespondierenden Plasmiden pSF-MDR, pMP-MDR etc. zu unterscheiden.

### Beispiel 3

### Konstruktion von replikationsfähigen Insertionsmutagenese-Vektoren basierend auf den erfindungsgemäßen Friend-/MESV-Vektorkonstrukten

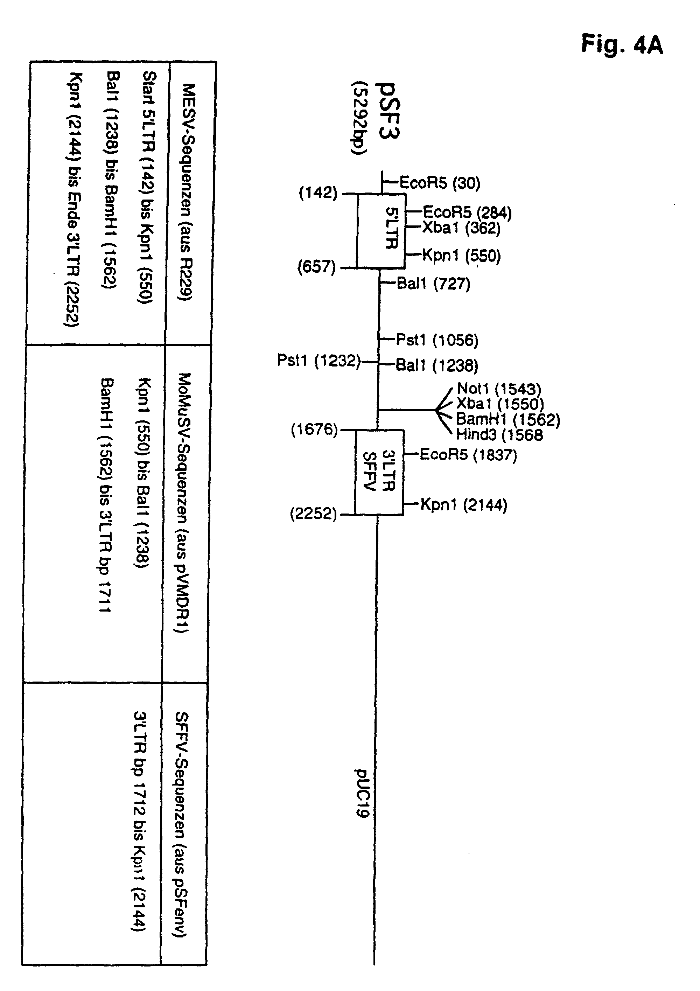

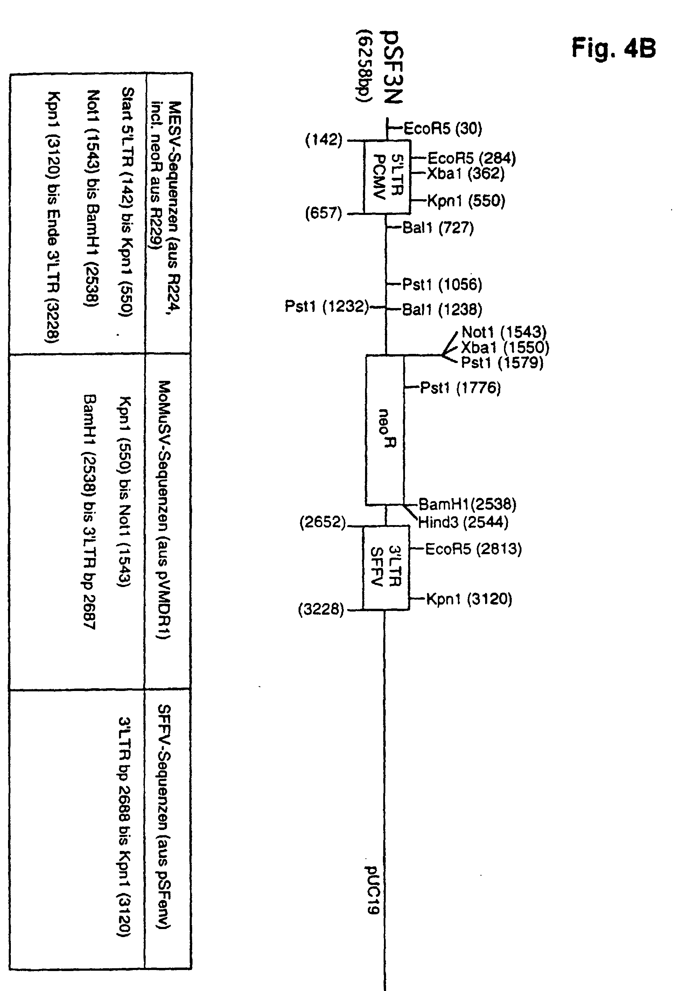

Das Beispielkonstrukt eines replikationsfähigen, amphotropen Friend-/MESV-Vektors zur Insertionsmutagenese wurde durch Klonierung der zur retroviralen Replikation notwendigen gag-pol-env-Gene in dem Friend-/MESV-Vektor pSF1 und pSF3 (siehe Fig. 2A und 4A) hergestellt.

Dazu wurde die gesamte 7,2 kb umfassende gag-pol-env-Genkassette aus pAM (Miller et al. (1985) *(35)*) mit Hilfe des "Expand long template PCR Systems" (Boehringer Mannheim, siehe auch: W.M. Barns (1994) *(36)*) amplifiziert und in pSF1 und pSF3 eingebaut.

pAM stellt ein Fusionskonstrukt aus dem ecotropen MoMuLV-Vektor pMLV-K (gag-pol bis SalI-Schnittstelle) und aus dem amphotropen Virus 4070A-Vektor p4070A (pol ab SalI-Schnittstelle und env^{amphotrop}) dar. Der obere PCR-Primer wurde 60 bp stromaufwärts des gag-Startcodons (direkt angrenzend an PstI-Schnittstelle), der untere PCR Primer direkt ans Ende des env^{amphotrop}-Gens gelegt. Das 7,2 kb gag-pol-env^{amphotrop} PCR-Amplifikat wurde ungeschnitten, d.h. mit stumpfen Enden, in den mit PstI und HindIII geschnittenen und zur Beseitigung der Überhänge mit Klenow-Enzym behandelten (Sambrook et al. (1989) *(37)*) Vektor pSF1 und pSF3 eingebaut. Die resultierenden Vektoren erhielten die Bezeichnungen pSF1-AM, pSF3-AM und haben folgenden prinzipiellen Aufbau: 5'-LTR^{MESV}-PBS(-)^{MESV}-gag-pol-env^{amphotrop}-3'-LTR^{SFFV} (pSF1-AM) und 5'-LTR^{MESV}-PBS(-)^{MUSV}-gag-pol-env^{anphotrop}-3'-LTR^{-SFFV}(pSF3-AM). Nach Transfektion in humane oder murine Zellen sind sie in der Lage, replikationskompetente Retroviren (RCR) zu bilden, die zur Insertionsmutagenese in hämatopoietischen Stamm- und Progenitorzellen eingesetzt werden können.

### Beispiel 4

### Konstruktion von replikationsfähigen Insertionsmutagenese-Vektoren basierend auf den erfindungsgemäßen MPSV/MESV-Vektorkonstrukten

Im Vektor MoMuLV-TAT (Hilberg, F. et al., Proc. Natl. Acad. Sci. USA 84 (1987), 5232 -5236) *(38)* wird das NheI-KpnI-Fragment durch das entsprechende LTR-Fragment von MPSV (-416 - +31) ersetzt. Der entstehende Vektor wird mit MP-CAT bezeichnet. Dieses Plasmid kann als Reportergenplasmid verwendet werden, indem die Expression des Chlorampenicol-Transferase (CAT)Gens unter der Kontrolle des retroviralen LTR-Fragments erfolgt.

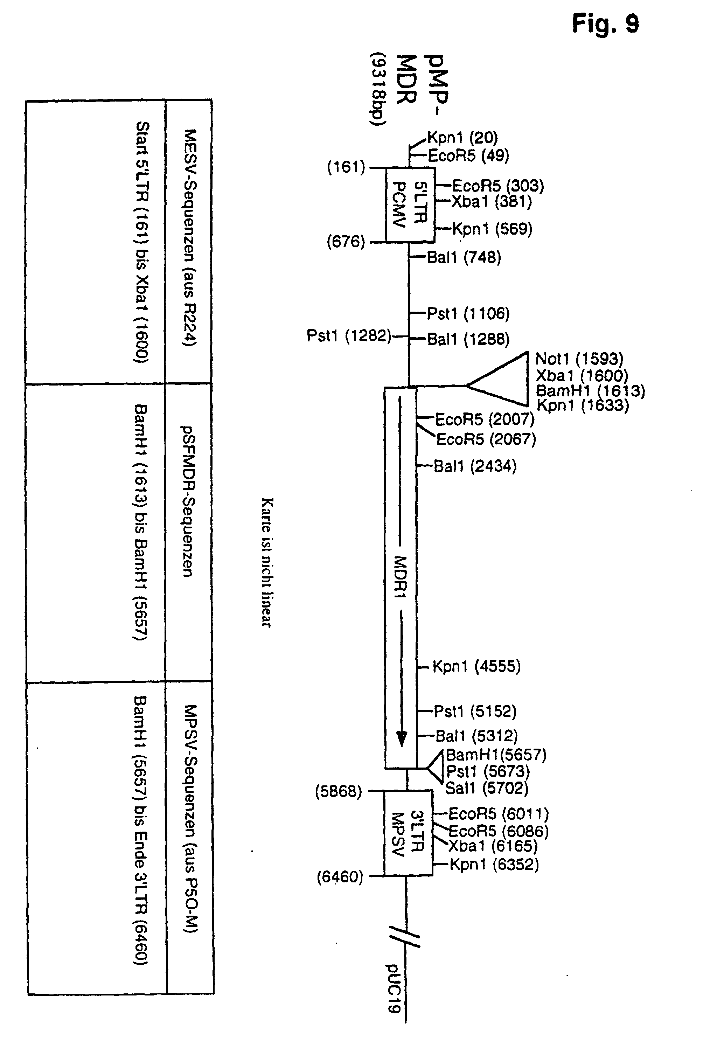

Als MPSV/MESV-Basisvektor wird der Vektor p50-M verwendet. Dieser Vektor enthält das MPSV-U3 im 3'-LTR. Er ist ein Derivat von p5Gneo, welches ein 537 bp großes Leaderfragment von d1587rev enthält (Grez M. et al., Proc. Natl. Acad. Sci. USA 87 (1990), 9202 - 9206 *(39)*, Colicelli J. und Goff S.P., J. Virol. 57 (1987) 37 - 45 *(40)).* NeoR-env codierende Regionen von p5Gneo wurden durch einen Polylinker aus p Bluescript KS (Stratagene) ersetzt, und das AUG von gag wurde durch Punktmutation zerstört. Die mdr-1 cDNA wurde als SacI/SspI-Fragment aus pMDR2000XS (Basenpaar -138 - +3878) (Chen C. et al., Cell 47 (1986) 381- 389 *(41)*) ausgeschnitten. Nach Subcloning in p Bluescript II KS wurde die cDNA als BamHI-Fragment isoliert und p50-M eingefügt. Der so hergestellte Vektor wird mit pMP-MDR bezeichnet. Die Sequenz von p-MDR, pMP-MDR ist in Fig. 9 und SEQ ID NO:6 dargestellt.

### Referenzliste

- (1): Miller A.D., Nature 357, 455-460.(1992)
- (2): Muffigan R.C., Science 260, 926-932 (1993)
- (3): Vile R. and Russell S.J., Gene Therapy 1, 88-98 (1994)
- (4): Lu M. et al., Human Gene Therapy 5, 203-208 (1994)
- (5): Palmer T.D. et al., Proc. Natl. Acad. Sci. U.S.A. 88, 1330-1334(1991)
- (6): Brenner M.K. et al., The Lancet 342, 1134-1137(1993)
- (7): Linemeyer D.L. et al., Proc. Natl. Acad. Sci. U.S.A. 78, 1401-1405 (1981)
- (8): R. Petersen et al., Mol. Cell. Biol. 11, 1213-1221 (1991)
- (9): Friel J. et al., J. Virol. 64, 369-378 (1990)
- (10): Clark S-P. and Mak T.W., NucL Acid Res. 10, 3315-3330 (1982)
- (11): Wolff L. et al., J. Virol. 53, 570-578 (1985)
- (12): Bestwick R.K. et al., J. Virol. 51, 695-705 (1984)
- (13): Koch W. et al., J. Virol. 49, 828-840 (1984)
- (14): Adachi A. et al., J. Virol. 50, 813-821 (1984)
- (15): Ostertag W. et al., Adv. Cancer Res. 48, 193-355 (1987)
- (16): McKnight S. and Tijan R., Cell 46, 795-805 (1986)
- (17): Ostertag W. et al., J. Virol. 33, 573-582 (1980)
- (18): Stocking C. et al., Proc. NatL Acad. Sci. U.S.A. 82, 5746-5750 (1985)
- (19): Franz T. et al., Proc. Natl. Acad. Sci. U.S.A. 83, 3292-3296 (1986)
- (20): Grez M. et al., J. Virol. 65, 4691-4698 (1991)
- (21): Stocking C. et al., in Virus Strategies, ed. by W. Doerfler and P. Böhm, VCH Verlagsgesellschaft, Weinheim, Germany (1993)
- (22): Grez M. et al., Proc. Natl. Acad. Sci. U.S.A. 87, 9202-9206 (1990)
- (23): Colicelli J. and Goff S.P., J. Virol. 57, 37-45 (1987)
- (24): Bilello J.A. et al., Virology 107, 331-344 (1980)
- (25): Pastan I. et al., Proc. Nat. Acad. Sci. USA 85, 4486-4490 (1988)
- (26): Ghattas I.R. et aL, MoL Cell. Biol. 11, 5848-5859 (1991)
- (27): Miller A.D. and Rosman G.J., Biotechniques 7, 980-990 (1990)
- (28): Anderson W.F., Human Gene Therapy 5, 1-2(1994)
- (29): Ohashi T. et al., Proc. Nat. Acad. Sci. USA 89, 11332-11336 (1992)
- (30): T. Kitamura et al., J. Cell. Physiol. 140, 323-334 (1989)
- (31): M Kriegler, Gene Transfer and Expression, A Laboratory Manual, W.H. Freeman & Co., New York (1990), 47-55 und 161-164
- (32): P. Artelt et al., Gene 99, 249-254 (1991)
- (33): D. Johnson et al., Cell 47, 545-554 (1986)
- (34): H.J. Kung et al., Current Topics in MicrobioL & Immunol. 171, 1-25 (1991)
- (35): A.D. Miller et al., Molecular and Cell Biology, Vol. 5, No. 3, 431-437 (1985)
- (36): W.M. Barns, Proc. Natl. Acad. Sci. USA 91, 2216-2220(1994)
- (37): J. Sambrook et al., Molecular Cloning: A Laboratory Manual, second edition (1989), CSH Laboratory Press
- (38): Hilberg, F. et al, Proc. Natl. Acad. Sci. USA 84, 5232-5236(1987)
- (39): Grez M. et al., Proc. Natl. Acad. Sci. USA 87, 9202-9206 (1990)
- (40): Colicelli J. und Goff S.P., J. Virol. 57, 37-45(1987)
- (41): Chen C. et al., Cell 47, 381-389(1986)
- (42): Velu T.J. et al., Colloque ISERM (Institut National de la Santé et de la Recherche Médicale), Vol. 219, Transfer de Genes chez l'Homme; (ISERM (National Institute of Health and Medical Research Colloquium), Vol. 219, Human Gene Transfer, (1991) 273-274, ed. Cohen-Haguenauer O. & Boiron, M.

## Patentansprüche

1. Retrovirales Vektorhybrid, **dadurch gekennzeichnet, daß** es
a) in der Leaderregion als U5-Region und tRNA primer binding site die U5-Region und die tRNA primer binding site Region von MESV und
b) als U3- und R-Region in 3'-LTR die U3- und R-Regionen aus Friend murine leukemia virus (F-MuLV) oder aus Myeloproliferative Sarkomavirus (MPSV)
enthält.

2. Vektorhybrid nach Anspruch 1, **dadurch gekennzeichnet, daß** als 5'-LTR bp 142 - 657 aus SEQ ID NO:1 und/oder als 3'-LTR bp 1707 - 2283 aus SEQ ID NO:1 enthält.

3. Vektorhybrid nach Anspruch 1, **dadurch gekennzeichnet, daß** es aus folgender Gruppe ausgewählt ist:
pSF1, das die Sequenz gemäß SEQ ID NO:1 aufweist;
pSF2, das die Sequenz gemäß SEQ ID NO:2 aufweist;
pSF3, das die Sequenz gemäß SEQ ID NO:3 aufweist;
pMH1, das die Sequenz gemäß SEQ ID NO:4 aufweist; und
pMP-MDR, das die Sequenz gemäß SEQ ID NO:6 aufweist.

4. Verfahren zur Herstellung eines replikationsdefekten, infektiösen Viruspartikels durch Transfektion einer eukaryontischen Helferzelle, welche die Helferfunktionen gag, env und pol besitzt, mit einem Vektorhybrid, welches in der Leaderregion als U5-Region und tRNA primer binding site die U5-Region und tRNA primer binding site von MESV und als U3- und R-Regionen in 3'-LTR die U3- und R-Regionen aus Friend murine leukemia virus (F-MuLV) oder Myeloproliferative Sarkomvirus (MPSV) enthält, Produktion der DNA des Vektorhybrids entsprechender RNA als Virusgenom in der Zelle, Verpackung des Virusgenoms in in der Zelle entstandene replikationsdefiziente, leere Virushüllen und Isolation der infektiösen Viruspartikel, welche das genannte Virusgenom enthalten.

5. Replikationsdefektes infektiöses Viruspartikel, erhältlich durch ein Verfahren nach Anspruch 4.

6. Verfahren zur Herstellung einer retroviral transduzierten eukaryontischen Zelle wobei humane embryonale Stommzellen ausgeschlossen sind, welche ein aktives exogenes Gen enthält, **dadurch gekennzeichnet, daß** die eukaryontische Zelle mit einem retroviralen Vektorvirus transduziert wird, der in seinem Genom
a) in der Leaderregion als U5-Region und tRNA primer binding site die US-Region und die tRNA primer binding site von MESV,
b) als U3- und R-Regionen in 3'-LTR die U3- und R-Regionen aus Friend murine leukemia virus (F-MuLV) oder Myeloproliferative Sarkomavirus (MPSV) und
c) das genannte exogene Gen enthält.

7. Retroviral transduzierte, eukaryontische Zelle erhältlich durch ein Verfahren nach Anspruch 6, wobei humane embryonale Stommzellen ausgeschlossen sind.

8. Verwendung eines replikationsdefekten retroviralen Vektorhybrids nach Anspruch 1 bis 3 zur Herstellung eines Arzneimittels zur ex vivo- oder in vivo-Gentherapie.

## Claims

1. Retroviral vector hybrid, **characterised in that** it contains
a) the U5 region and the tRNA primer binding site region of MESV in the leader region as U5 region and tRNA primer binding site and
b) the U3 and R regions from Friend murine leukaemia virus (F-MuLV) or from myeloproliferative sarcoma virus (MPSV) as U3 and R region in 3' LTR.

2. Vector hybrid according to Claim 1, **characterised in that** [it] contains bp 142 - 657 from SEQ ID NO:1 as 5' LTR and/or bp 1707 - 2283 from SEQ ID NO:1 as 3' LTR.

3. Vector hybrid according to Claim 1, **characterised in that** it is selected from the following group:
pSF1, which has the sequence according to SEQ ID NO:1;
pSF2, which has the sequence according to SEQ ID NO:2;
pSF3, which has the sequence according to SEQ ID NO:3;
pMH1, which has the sequence according to SEQ ID NO:4; and
pMP-MDR, which has the sequence according to SEQ ID NO: 6.

4. Process for the production of a replication-defective, infectious virus particle by transfection of a eukaryotic helper cell, which possesses the helper functions gag, env and pol, with a vector hybrid which contains the U5 region and tRNA primer binding site of MESV in the leader region as tU5 region and tRNA primer binding site and the U3 and R regions from Friend murine leukaemia virus (F-MuLV) or myeloproliferative sarcoma virus (MPSV) as U3 and R regions in 3' LTR, production of RNA corresponding to the DNA of the vector hybrid as viral genome in the cell, packaging of the viral genome in replication-deficient, empty viral envelopes formed in the cell and isolation of the infectious virus particles which contain the said viral genome.

5. Replication-defective infectious virus particle obtainable by a process according to Claim 4.

6. Process for the production of a retrovirally transduced eukaryotic cell, excluding human embryonic stem cells, which contains an active exogenous gene, **characterised in that** the eukaryotic cell is transduced with a retroviral vector virus which contains in its genome
a) the U5 region and the tRNA primer binding site of MESV in the leader region as U5 region and tRNA primer binding site,
b) the U3 and R regions from Friend murine leukaemia virus (F-MuLV) or myeloproliferative sarcoma virus (MPSV) as U3 and R regions in 3' LTR and
c) the said exogenous gene.

7. Retrovirally transduced, eukaryotic cell obtainable by a process according to Claim 6, human embryonic stem cells being excluded.

8. Use of a replication-defective retroviral vector hybrid according to Claim 1 to 3 for the production of a medicament for ex vivo or in vivo gene therapy.

## Revendications

1. Vecteur rétroviral hybride, **caractérisé en ce qu'**il comprend :
a) dans la séquence de tête, en tant que région U5 et site de liaison de l'amorce d'ARNt, la région U5 et la région du site de liaison de l'amorce d'ARNt de MESV et
b) en tant que régions U3 et R en 3'-LTR, les régions U3 et R provenant du virus de la leucémie murine de Friend (F-MuLV) ou du virus du sarcome myéloprolifératif (MPSV).

2. Vecteur hybride selon la revendication 1, **caractérisé en ce qu'**il comprend, en tant que 5'-LTR, les paires de bases 142 - 657 provenant de la SEQ ID N°1 et/ou, en tant que 3'-LTR, les paires de bases 1707 - 2283 provenant de la SEQ ID N°1.

3. Vecteur hybride selon la revendication 1, **caractérisé en ce qu'**il est choisi parmi le groupe suivant :
pSF1, présentant la séquence conformément à la SEQ ID N°1;
pSF2, présentant la séquence conformément à la SEQ ID N°2;
pSF3, présentant la séquence conformément à la SEQ ID N°3;
pMH1, présentant la séquence conformément à la SEQ ID N°4; et
pMP-MOR, présentant la séquence conformément à la SEQ ID N°6.

4. Procédé de préparation d'une particule virale infectieuse défective pour la réplication, par transfection d'une cellule helper eucaryote qui possède les fonctions helper gag, env et pol, avec un vecteur hybride qui comprend dans la séquence de tête, en tant que région U5 et site de liaison de l'amorce d'ARNt, la région U5 et le site de liaison de l'amorce d'ARNt de MESV et, en tant que régions U3 et R en 3'-LTR, les régions U3 et R du virus de la leucémie murine de Friend (F-MuLV) ou du virus du sarcome myéloprolifératif (MPSV), de production dans la cellule d'ADN du vecteur hybride correspondant à l'ARN du génome viral, d'empaquetage dans des enveloppes virales vides du génome viral défectif pour la réplication, apparu dans la cellule, et d'isolation des particules virales infectieuses qui comprennent le génome viral cité.

5. Particules virales infectieuses, défectives pour la réplication, susceptibles d'être obtenues par un procédé selon la revendication 4.

6. Procédé de préparation d'une cellule eucaryote, dont sont exclues les cellules souches d'embryons humains, ayant subi une transduction rétrovirale, comprenant un gène exogène actif, **caractérisé en ce que** la cellule eucaryote est transduite par un vecteur rétroviral qui comprend dans son génome :
a) dans la séquence de tête, en tant que région U5 et site de liaison de l'amorce d'ARNt, la région U5 et la région du site de liaison de l'amorce d'ARNt de MESV et
b) en tant que régions U3 et R en 3'-LTR, les régions U3 et R provenant du virus de la leucémie murine de Friend (F-MuLV) ou du virus du sarcome myéloprolifératif (MPSV) et
c) le gène exogène cité.

7. Cellule eucaryote, dont sont exclues les cellules souches d'embryons humains, transduite par un rétrovirus, susceptible d'être obtenue par un procédé selon la revendication 6.

8. Utilisation d'un vecteur hybride rétroviral défectif pour la réplication, selon la revendication 1 à 3, pour la préparation d'un médicament pour la thérapie génique ex vivo ou in vivo.
